# EUROPEAN PATENT APPLICATION

(11) **EP 4 685 150 A1**
(43) Date of publication of application: **28.01.2026**
(21) Application number: 24797122.9
(22) Date of filing: 25.04.2024
(51) Int. Cl.: C07K 1/06, C07D 263/44

(54) **METHOD FOR PRODUCING AMIDE GROUP-CONTAINING COMPOUND USING N-CARBOXYAMINO ACID ANHYDRIDE**

(30) Priority: 28.04.2023 JP 2023074719
(71) Applicant: CHUGAI SEIYAKU KABUSHIKI KAISHA, Tokyo 115-8543 (JP)
(72) Inventor: HOU, Zengye, Tokyo 115-8543 (JP); KOMIYA, Shio, Tokyo 115-8543 (JP)
(74) Representative: Vossius & Partner Patentanwälte Rechtsanwälte mbB
(86) International application number: PCT/JP2024/016239
(87) International publication number: WO 2024/225377

(57) **Abstract**

A method for producing an amide group-containing compound, comprising step A of reacting NCA with an amino group of an amino group-containing compound in a first solvent in the presence of a Lewis acid.

## Description

### Technical Field

The present invention relates to a method for producing an amide group-containing compound using an amino acid N-carboxyanhydride.

### Background Art

Peptides are molecules in which a plurality of amino acids are linked. Peptides play an indispensable role in the life activities of the organisms. In general, peptides are chemically synthesized by extending the peptide chain by repeating a protection of functional groups other than those involved in the condensation reaction of amino acids, a coupling of protected amino acids to each other, and a deprotection.

On the other hand, there is a known method for elongating a peptide chain without requiring the deprotection by reacting an N-carboxyanhydride (NCA) with an amino acid whose carboxy group is protected. However, a problem of this method is that over-elongation products in which two or more NCAs are consecutively reacted, are generated and the selectivity is reduced.

To overcome this problem, various methods for suppressing over-elongation reactions have been studied. For example, Non Patent Literature 1 proposes a method for strictly controlling pH using a continuous stirred tank reactor (CSTR) in an aqueous solvent; Non Patent Literature 2 proposes a method for controlling the reaction under cryogenic (-50 to -40°C) and anhydrous conditions; and Non Patent Literature 3 proposes a method for controlling the reaction under reaction conditions of ball milling with addition of an inorganic base.

### Citation List

### Patent Literature

Patent Literature 1: International Publication No. WO 2014/052537

### Non Patent Literature

Non Patent Literature 1: Org. ProcessRes. Dev. 2017, 21, 1557-1565.
Non Patent Literature 2: J. Chem. Soc., 1950, 3461-3466.
Non Patent Literature 3: Sustainable Chemistry and Pharmacy 2021, 24, 100540.

### Summary of Invention

### Technical Problem

However, in the methods of Non Patent Literatures 1 to 3, there are problems such as limitation of applicable substrates and difficulty in practical application.

Accordingly, it is an object of the present invention to provide a method for producing an amide group-containing compound using NCA in which the generation of over-elongation products is sufficiently suppressed.

### Solution to Problem

In view of the above circumstances, the present inventors have intensively studied and finally completed the present invention.

For example, the present invention provides the following [A1] to [A28].
[A1] A method for producing an amide group-containing compound, including step A of reacting an N-carboxyanhydride(NCA) with an amino group of an amino group-containing compound in a first solvent in the presence of a Lewis acid.
[A2] The method according to [A1], wherein the Lewis acid is a metal salt.
[A3] The method according to [A2], wherein a metal ion of the metal salt is at least one selected from the group consisting of a calcium ion, a zinc ion, a lanthanum ion, a terbium ion, a holmium ion, a thulium ion, a scandium ion, a bismuth ion, an iron ion, a barium ion, a cerium ion, a neodymium ion, a samarium ion, an europium ion, a gadolinium ion, an erbium ion, a lutetium ion, an ytterbium ion, an indium ion, a vanadium ion, a hafnium ion, and a strontium ion.
[A3-1] The method according to [A2], wherein a metal ion of the metal salt is at least one selected from the group consisting of a calcium ion, a zinc ion, a lanthanum ion, a terbium ion, a holmium ion, a thulium ion, a scandium ion, a bismuth ion, an iron ion, a barium ion, a cerium ion, a neodymium ion, a samarium ion, an europium ion, a gadolinium ion, an erbium ion, and a lutetium ion.
[A3-2] The method according to [A2], wherein a metal ion of the metal salt is at least one selected from the group consisting of a calcium ion, a zinc ion, a lanthanum ion, a terbium ion, a holmium ion, and a thulium ion.
[A3-3] The method according to [A2], wherein a metal ion of the metal salt is a calcium ion or a lanthanum ion.
[A4] The method according to [A2] or [A3], wherein an anion of the metal salt is at least one selected from the group consisting of a trifluoromethanesulfonate ion, an iodide ion, a bromide ion, a chloride ion, and a nitrate ion.
[A4-1] The method according to [A2] or [A3], wherein an anion of the metal salt is at least one selected from the group consisting of a trifluoromethanesulfonate ion and an iodide ion.
[A5] The method according to [A2], wherein the metal salt is at least one selected from the group consisting of calcium trifluoromethanesulfonate, calcium iodide, zinc trifluoromethanesulfonate, lanthanum(III) trifluoromethanesulfonate, terbium(III) trifluoromethanesulfonate, holmium(III) trifluoromethanesulfonate, thulium(III) trifluoromethanesulfonate, scandium(III) trifluoromethanesulfonate, bismuth(III) trifluoromethanesulfonate, iron(II) trifluoromethanesulfonate, barium(II) trifluoromethanesulfonate, lanthanum(III) chloride bis(lithium chloride) complex, cerium(III) trifluoromethanesulfonate, neodymium(III) trifluoromethanesulfonate, samarium(III) trifluoromethanesulfonate, europium(III) trifluoromethanesulfonate, gadolinium(III) trifluoromethanesulfonate, erbium(III) trifluoromethanesulfonate, lutetium(III) trifluoromethanesulfonate, ytterbium(III) trifluoromethanesulfonate, zinc chloride, indium(III) bromide, vanadium(III) chloride, hafnium(IV) trifluoromethanesulfonate, lanthanum(III) nitrate hexahydrate, and strontium(II) trifluoromethanesulfonate. [A5-1] The method according to [A2], wherein the metal salt is at least one selected from the group consisting of calcium trifluoromethanesulfonate, calcium iodide, zinc trifluoromethanesulfonate, lanthanum(III) trifluoromethanesulfonate, terbium(III) trifluoromethanesulfonate, holmium(III) trifluoromethanesulfonate, thulium(III) trifluoromethanesulfonate, scandium(III) trifluoromethanesulfonate, bismuth(III) trifluoromethanesulfonate, iron(II) trifluoromethanesulfonate, barium(II) trifluoromethanesulfonate, lanthanum(III) chloride bis(lithium chloride) complex, cerium(III) trifluoromethanesulfonate, neodymium(III) trifluoromethanesulfonate, samarium(III) trifluoromethanesulfonate, europium(III) trifluoromethanesulfonate, gadolinium(III) trifluoromethanesulfonate, erbium(III) trifluoromethanesulfonate, and lutetium(III) trifluoromethanesulfonate.
[A5-2] The method according to [A2], wherein the metal salt is at least one selected from the group consisting of calcium trifluoromethanesulfonate, calcium iodide, zinc trifluoromethanesulfonate, lanthanum(III) trifluoromethanesulfonate, terbium(III) trifluoromethanesulfonate, holmium(III) trifluoromethanesulfonate, and thulium(III) trifluoromethanesulfonate.
[A5-3] The method according to [A2], wherein the metal salt is calcium iodide or lanthanum(III) trifluoromethanesulfonate.
[A6] The method according to any one of [A1] to [A5], wherein the Lewis acid is a hard Lewis acid or a borderline hardness Lewis acid according to the HSAB theory.
[A6-1] The method according to any one of [A1] to [A5], wherein the Lewis acid is a hard Lewis acid according to the HSAB theory.
[A7] The method according to any one of [A1] to [A6], wherein the NCA is a compound represented by the following formula (1): 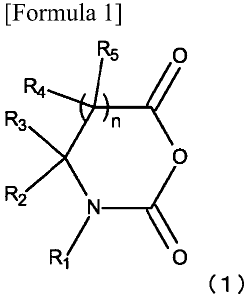
   wherein n represents 0 or 1;
      (i) R₁ is hydrogen or C₁-C₈ alkyl, or
      (ii) R₁ and R₂, together with an atom to which they are bonded, form a 4- to 7-membered saturated heterocycle;
   in the case of (i), R₂, R₃, R₄ and R₅ are each independently selected from the group consisting of hydrogen, C₁-C₆ alkyl, C₃-C₈ cycloalkyl, C₆-C₁₀ aryl C₁-C₆ alkyl, and 5- to 10-membered heteroaryl C₁-C₆ alkyl, each of which may be substituted by one or more groups independently selected from the group consisting of halogen, hydroxy, C₁-C₃ alkyl, halo C₁-C₆ alkyl, and C₁-C₆ alkoxycarbonyl, or
   R₂ and R₃, or R₄ and R₅, together with an atom to which they are each bonded, may form a 3- to 8-membered alicyclic ring or a 4- to 7-membered saturated heterocycle;
   in the case of (ii), R₃, R₄, and R₅ are each independently selected from the group consisting of hydrogen, C₁-C₆ alkyl, C₃-C₈ cycloalkyl, C₆-C₁₀ aryl C₁-C₆ alkyl, and 5- to 10-membered heteroaryl C₁-C₆ alkyl, each of which may be substituted by one or more groups independently selected from the group consisting of halogen, hydroxy, C₁-C₃ alkyl, halo C₁-C₆ alkyl, and C₁-C₆ alkoxycarbonyl, or R₄ and R₅, together with an atom to which they are each bonded, may form a 3- to 8-membered alicyclic ring or a 4- to 7-membered saturated heterocycle.
[A7-1] The method according to [A7], wherein n is 0.
[A8] The method according to [A7], wherein R₁ is hydrogen or C₁-C₆ alkyl.
[A8-1] The method according to [A7], wherein R₁ is hydrogen or C₁-C₃ alkyl.
[A8-2] The method according to [A7], wherein R₁ is methyl or ethyl.
[A8-3] The method according to [A7], wherein R₁ is methyl.
[A9] The method according to [A7] or [A8], wherein R₂, R₃, R₄ and R₅ are each independently selected from the group consisting of hydrogen, C₁-C₆ alkyl, and C₆-C₁₀ aryl C₁-C₆ alkyl, each of which may be substituted by one or more groups independently selected from the group consisting of hydroxy, C₁-C₃ alkyl, halo C₁-C₆ alkyl, and C₁-C₆ alkoxycarbonyl.
[A9-1] The method according to [A7] or [A8], wherein R₂, R₃, R₄ and R₅ are each independently selected from the group consisting of hydrogen, C₁-C₆ alkyl, and C₆-C₁₀ aryl C₁-C₆ alkyl.
[A10] The method according to [A7], wherein n is 0; R₁ is hydrogen or C₁-C₈ alkyl;
   R₂ and R₃ are each independently selected from the group consisting of hydrogen, C₁-C₆ alkyl, and C₆-C₁₀ aryl C₁-C₆ alkyl, each of which may be substituted by one or more groups independently selected from the group consisting of hydroxy, C₁-C₃ alkyl, halo C₁-C₆ alkyl, and C₁-C₆ alkoxycarbonyl.
[A10-1] The method according to [A7], wherein n is 0; R₁ is hydrogen or C₁-C₆ alkyl;
   R₂ and R₃ are each independently selected from the group consisting of hydrogen, C₁-C₆ alkyl, and C₆-C₁₀ aryl C₁-C₆ alkyl.
[A10-2] The method according to [A7], wherein n is 0, R₁ is methyl, R₂ is hydrogen, and R₃ is selected from the group consisting of methyl, ethyl, and benzyl.
[A11] The method according to any one of [A1] to [A10], wherein the NCA is selected from the group consisting of (S)-3,4-dimethyloxazolidine-2,5-dione, (S)-4-benzyl-3-methyloxazolidine-2,5-dione, 3-butyloxazolidine-2,5-dione, (S)-4-ethyl-3-methyloxazolidine-2,5-dione, (S)-4-methyloxazolidine-2,5-dione, (S)-4-isopropyloxazolidine-2,5-dione, and (S)-4-hydroxybenzyloxazolidine-2,5-dione.
[A12] The method according to any one of [A1] to [A11], wherein the amino group-containing compound is selected from the group consisting of a peptide, an amino acid, an amino acid amide, and an amine.
[A13] The method according to any one of [A1] to [A12], wherein the amino group of the amino group-containing compound is a primary amino group or a secondary amino group.
[A14] The method according to any one of [A1] to [A13], wherein the amino group-containing compound is a compound selected from the group consisting of a peptide, an amino acid, and an amino acid amide.
[A15] The method according to any one of [A12] to [A14], wherein, in the compound selected from the group consisting of a peptide, an amino acid, and an amino acid amide, an amino group at the N-terminus is an amino group involved in a reaction of the step A.
[A16] The method according to any one of [A12] to [A15], wherein the compound selected from the group consisting of a peptide, an amino acid, and an amino acid amide is any of the following (a) to (c):
   (a) a compound in which the amino group reacting with the NCA is a primary amino group;
   (b) a compound in which the amino group reacting with the NCA is a secondary amino group, and a carbon atom to which the amino group reacting with the NCA is directly bonded is not substituted, wherein the carbon atom is a carbon atom in a main chain of an amino acid residue;
   (c) a compound in which the amino group reacting with the NCA is a secondary amino group, and a 4- to 7-membered saturated heterocycle containing a nitrogen atom of the secondary amino group and a carbon atom to which the amino group reacting with the NCA is directly bonded is formed, wherein the carbon atom is a carbon atom in a main chain of an amino acid residue.
[A16-1] The method according to any one of [A12] to [A15], wherein the compound selected from the group consisting of a peptide, an amino acid, and an amino acid amide is (a) a compound in which the amino group reacting with the NCA is a primary amino group.
[A16-2] The method according to any one of [A12] to [A15], wherein the compound selected from the group consisting of a peptide, an amino acid, and an amino acid amide is (b) an amino group-containing compound in which the amino group reacting with the NCA is a secondary amino group, and a carbon atom to which the amino group reacting with the NCA is directly bonded is not substituted, wherein the carbon atom is a carbon atom in a main chain of an amino acid residue.
[A16-3] The method according to any one of [A12] to [A15], wherein the compound selected from the group consisting of a peptide, an amino acid, and an amino acid amide is (c) a compound in which the amino group reacting with the NCA is a secondary amino group, and a 4- to 7-membered saturated heterocycle containing a nitrogen atom of the secondary amino group and a carbon atom to which the amino group reacting with the NCA is directly bonded is formed, wherein the carbon atom is a carbon atom in a main chain of an amino acid residue.
[A17] The method according to any one of [A1] to [A16], wherein the amino group-containing compound is a peptide.
[A17-1] The method according to [A17], wherein the peptide is composed of 2 to 20 amino acid residues.
[A17-2] The method according to [A17], wherein the peptide is selected from the group consisting of tert-butyl L-phenylalaninate, tert-butyl (S)-3-((S)-2-amino-N-methyl-3-phenylpropanamide)-4-(dimethylamino)-4-oxobutanoate, benzyl L-isoleucinate, tert-butyl N-((S)-N-ethyl-azetidine-2-carboxamide)-3-(p-tolyl)propanoyl)-N-methylglycinate.
[A18] The method according to any one of [A1] to [A17], wherein the amino group-containing compound is an amine.
[A18-1] The method according to [A18], wherein the amine is selected from the group consisting of a C₁-C₈ alkylamine, a diC₁-C₈ alkylamine, and a compound containing a 4- to 7-membered saturated heterocycle containing a nitrogen atom of a secondary amino group.
[A18-2] The method according to [A18], wherein the amine is selected from the group consisting of methylamine, ethylamine, dimethylamine, diethylamine, morpholine, piperidine, and pyrrolidine.
[A18-3] The method according to [A18], wherein the amine is selected from the group consisting of dimethylamine, diethylamine, morpholine, piperidine, and pyrrolidine.
[A19] The method according to any one of [A1] to [A18], wherein the first solvent is a solvent containing at least one selected from the group consisting of 2-methyltetrahydrofuran, toluene, tetrahydrofuran, 4-methyltetrahydropyran, isopropyl acetate, acetonitrile, and 1,3-dimethyl-2-imidazolidinone.
[A19-1] The method according to any one of [A1] to [A18], wherein the first solvent is a solvent containing at least one selected from the group consisting of 2-methyltetrahydrofuran, toluene, tetrahydrofuran, 4-methyltetrahydropyran, and isopropyl acetate.
[A19-2] The method according to any one of [A1] to [A18], wherein the first solvent is a solvent containing at least one selected from the group consisting of 2-methyltetrahydrofuran and toluene.
[A20] The method according to any one of [A1] to [A19], wherein, in the step A, the Lewis acid is 0.1 molar equivalents to 5.0 molar equivalents relative to the amino group-containing compound.
[A20-1] The method according to any one of [A1] to [A19], wherein, in the step A, the Lewis acid is 0.25 molar equivalents to 2.0 molar equivalents relative to the amino group-containing compound.
[A20-2] The method according to any one of [A1] to [A19], wherein, in the step A, the Lewis acid is 0.25 molar equivalents to 1.0 molar equivalent relative to the amino group-containing compound.
[A20-3] The method according to any one of [A1] to [A19], wherein, in the step A, the Lewis acid is 0.25 molar equivalents to 0.5 molar equivalents relative to the amino group-containing compound.
[A21] The method according to any one of [A1] to [A20], wherein, in the step A, the NCA is 0.8 molar equivalents to 5.0 molar equivalents relative to the amino group-containing compound.
[A21-1] The method according to any one of [A1] to [A20], wherein, in the step A, the NCA is 1.0 molar equivalent to 2.0 molar equivalents relative to the amino group-containing compound.
[A21-2] The method according to any one of [A1] to [A20], wherein, in the step A, the NCA is 1.1 molar equivalents to 1.5 molar equivalents relative to the amino group-containing compound.
[A22] The method according to any one of [A1] to [A21], wherein, in the step A, a liquid temperature of the first solvent is 0 to 40°C.
[A23] The method according to any one of [A1] to [A22], wherein, in the step A, a reaction time is 0.5 to 48 hours.
[A24] The method according to any one of [A1] to [A23], wherein the step A is carried out in the absence of a base.
[A25] The method according to any one of [A1] to [A24], wherein the step A is carried out in the absence of a desiccant.
[A26] The method according to any one of [A1] to [A25], wherein a nucleophilic reagent is not added to a reaction mixture after the step A.
[A27] The method according to any one of [A1] to [A26], wherein the step A is repeated two or more times.
[A28] The method according to any one of [A1] to [A27], wherein a peak area of an over-elongation product is reduced to 1% or less relative to the sum of peak areas of an amide group-containing compound into which one amino acid residue from NCA is introduced and the over-elongation product, as determined by UVArea% at 210 nm by HPLC analysis.

The present invention also provides, for example, the following [B1] to [B11].
[B1] The method according to any one of [A1] to [A28], including, prior to the step A, step B of contacting an amino acid protected with a Boc group with a first activator in a second solvent to produce the NCA.
[B2] The method according to [B1], wherein a reaction mixture containing the NCA obtained in the step B is used directly in the step A.
[B3] The method according to [B1], including, after the step B, step C of removing at least a portion of the second solvent and mixing with a third solvent.
[B3-1] The method according to [B3], wherein a solution containing the NCA obtained in the step C is used directly in the step A.
[B4] The method according to [B1], wherein the second solvent is removed from a reaction mixture obtained in the step B, and the resulting mixture containing the NCA is used without purification in the step A.
[B5] The method according to any one of [B1] to [B4], wherein the amino acid protected with a Boc group is a compound represented by the following formula (2): 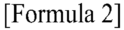 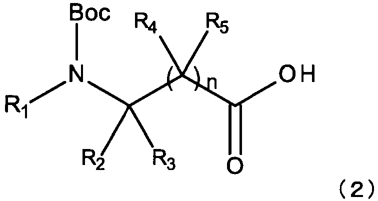 wherein n, R₁, R₂, R₃, R₄ and R₅ has the same meaning as n, R₁, R₂, R₃, R₄ and R₅ in formula (1), respectively.
[B6] The method according to any one of [B1] to [B5], wherein the second solvent is a solvent containing at least one selected from the group consisting of tetrahydrofuran, 2-methyltetrahydrofuran, isopropyl acetate, toluene, and acetonitrile.
[B6-1] The method according to any one of [B1] to [B5], wherein the second solvent is a solvent containing at least one selected from the group consisting of tetrahydrofuran, 2-methyltetrahydrofuran, and isopropyl acetate.
[B6-2] The method according to any one of [B1] to [B5], wherein the second solvent is tetrahydrofuran.
[B6-3] The method according to any one of [B1] to [B5], wherein the second solvent is a solvent containing at least one selected from the group consisting of tetrahydrofuran, 2-methyltetrahydrofuran, isopropyl acetate, ethyl acetate, toluene, and acetonitrile.
[B6-4] The method according to any one of [B1] to [B5], wherein the second solvent is a solvent containing at least one selected from the group consisting of tetrahydrofuran, 2-methyltetrahydrofuran, isopropyl acetate, and ethyl acetate.
[B6-5] The method according to any one of [B1] to [B5], wherein the second solvent is tetrahydrofuran or ethyl acetate.
[B7] The method according to any one of [B3] to [B6], wherein the third solvent is a solvent containing at least one selected from the group consisting of 2-methyltetrahydrofuran, toluene, tetrahydrofuran, 4-methyltetrahydropyran, isopropyl acetate, acetonitrile, and 1,3-dimethyl-2-imidazolidinone.
[B7-1] The method according to any one of [B3] to [B6], wherein the third solvent is a solvent containing at least one selected from the group consisting of 2-methyltetrahydrofuran, toluene, tetrahydrofuran, 4-methyltetrahydropyran, and isopropyl acetate.
[B7-2] The method according to any one of [B3] to [B6], wherein the third solvent is a solvent containing at least one selected from the group consisting of 2-methyltetrahydrofuran and toluene.
[B8] The method according to any one of [B3] to [B6], wherein the first solvent includes the same solvent as the third solvent.
[B8-1] The method according to any one of [B3] to [B6], wherein the first solvent is the same solvent as the third solvent.
[B9] The method according to any one of [B1] to [B8], wherein the first activator is at least one selected from the group consisting of thionyl chloride, oxalyl chloride, phosphorus trichloride, triphosgene, and dichloromethyl methyl ether.
[B9-1] The method according to any one of [B1] to [B8], wherein the first activator is at least one selected from the group consisting of thionyl chloride, oxalyl chloride, and phosphorus trichloride.
[B9-2] The method according to any one of [B1] to [B8], wherein the first activator is thionyl chloride.
[B9-3] The method according to any one of [B1] to [B8], wherein the first activator is at least one selected from the group consisting of thionyl chloride, oxalyl chloride, phosphorus trichloride, triphosgene, propylphosphonic anhydride, and dichloromethyl methyl ether.
[B9-4] The method according to any one of [B1] to [B8], wherein the first activator is at least one selected from the group consisting of thionyl chloride, oxalyl chloride, phosphorus trichloride, and propylphosphonic anhydride.
[B9-5] The method according to any one of [B1] to [B8], wherein the first activator is thionyl chloride and propylphosphonic anhydride.
[B10] The method according to any one of [B1] to [B9], wherein the step B is carried out in the presence of a base.
[B10-1] The method according to any one of [B1] to [B9], wherein a base is added after the step B.
[B11] The method according to [B10], wherein the base is at least one selected from the group consisting of DIPEA, cesium carbonate, sodium hydrogen carbonate, 1-methylpiperidine, 4-ethylmorpholine, N-methylmorpholine, 2,4,6-collidine, potassium carbonate, potassium hydrogen carbonate, magnesium oxide, calcium oxide, tripotassium phosphate, dipotassium hydrogen phosphate, and cesium hydrogen carbonate.
[B11-1] The method according to [B10], wherein the base is at least one selected from the group consisting of DIPEA, cesium carbonate, sodium hydrogen carbonate, potassium carbonate, calcium oxide, and cesium hydrogen carbonate.
[B11-2] The method according to [B10], wherein the base is at least one selected from the group consisting of cesium carbonate, potassium carbonate, calcium oxide, and cesium hydrogen carbonate.
[B11-3] The method according to [B10], wherein the base is calcium oxide.
[B11-4] The method according to [B10], wherein the base is at least one selected from the group consisting of DIPEA, cesium carbonate, sodium hydrogen carbonate, 1-methylpiperidine, 4-ethylmorpholine, N-methylmorpholine, 2,4,6-collidine, pyridine, potassium carbonate, potassium hydrogen carbonate, magnesium oxide, calcium oxide, tripotassium phosphate, dipotassium hydrogen phosphate, and cesium hydrogen carbonate.
[B11-5] The method according to [B10], wherein the base is at least one selected from the group consisting of DIPEA, cesium carbonate, sodium hydrogen carbonate, pyridine, potassium carbonate, calcium oxide, and cesium hydrogen carbonate.
[B11-6] The method according to [B10], wherein the base is at least one selected from the group consisting of cesium carbonate, pyridine, potassium carbonate, calcium oxide, and cesium hydrogen carbonate.
[B11-7] The method according to [B10], wherein the base is calcium oxide or pyridine.

The present invention also provides, for example, the following [B'1] to [B'11].
[B'1] The method according to any one of [A1] to [A28], including, prior to the step A, step B' of contacting an amino acid with a second activator in a second solvent to produce the NCA.
[B'2] The method according to [B'1], wherein a reaction mixture containing the NCA obtained in the step B' is used directly in the step A.
[B'3] The method according to [B'1], including, after the step B', step C of removing at least a portion of the second solvent and mixing with a third solvent.
[B'3-1] The method according to [B'3], wherein a solution containing the NCA obtained in the step C is used directly in the step A.
[B'4] The method according to [B'1], wherein the second solvent is removed from a reaction mixture obtained in the step B', and the resulting mixture containing the NCA is used without purification in the step A.
[B'5] The method according to any one of [B'1] to [B'4], wherein the amino acid is a compound represented by the following formula (2'): 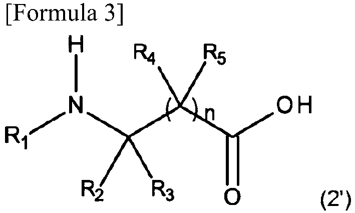 wherein n, R₁, R₂, R₃, R₄ and R₅ has the same meaning as n, R₁, R₂, R₃, R₄ and R₅ in formula (1), respectively.
[B'6] The method according to any one of [B'1] to [B'5], wherein the second solvent is a solvent containing at least one selected from the group consisting of tetrahydrofuran, 2-methyltetrahydrofuran, isopropyl acetate, toluene, and acetonitrile.
[B'6-1] The method according to any one of [B'1] to [B'5], wherein the second solvent is a solvent containing at least one selected from the group consisting of tetrahydrofuran, 2-methyltetrahydrofuran, and isopropyl acetate.
[B'6-2] The method according to any one of [B'1] to [B'5], wherein the second solvent is tetrahydrofuran.
[B'7] The method according to any one of [B'3] to [B'6], wherein the third solvent is a solvent containing at least one selected from the group consisting of 2-methyltetrahydrofuran, toluene, tetrahydrofuran, 4-methyltetrahydropyran, isopropyl acetate, acetonitrile, and 1,3-dimethyl-2-imidazolidinone.
[B'7-1] The method according to any one of [B'3] to [B'6], wherein the third solvent is a solvent containing at least one selected from the group consisting of 2-methyltetrahydrofuran, toluene, tetrahydrofuran, 4-methyltetrahydropyran, and isopropyl acetate.
[B'7-2] The method according to any one of [B'3] to [B'6], wherein the third solvent is a solvent containing at least one selected from the group consisting of 2-methyltetrahydrofuran and toluene.
[B'8] The method according to any one of [B'3] to [B'6], wherein the first solvent includes the same solvent as the third solvent.
[B'8-1] The method according to any one of [B'3] to [B'6], wherein the first solvent is the same solvent as the third solvent.
[B'9] The method according to any one of [B'1] to [B'8], wherein the second activator is a condensing agent having a carbonyl group.
[B'9-1] The method according to any one of [B'1] to [B'8], wherein the second activator is at least one selected from the group consisting of triphosgene, carbonyl imidazole, and di-tert-butyl dicarbonate.
[B'10] The method according to any one of [B'1] to [B'9], wherein the step B' is carried out in the presence of an acid.
[B'11] The method according to [B'10], wherein the acid is at least one selected from the group consisting of trifluoromethanesulfonic acid, methanesulfonic acid, and imidazole hydrochloride.

In the above numberings, the number cited in the dependent item includes the branch number of the number, unless otherwise mentioned. For example, the [A3] cited in the dependent item shows that not only [A3] but also its branch numbers [A3-1] to [A3-3] are included. The same applies to other numberings.

### Advantageous Effect of Invention

According to the present invention, a method for producing an amide group-containing compound using NCA in which the over-elongation reaction is sufficiently suppressed, can be provided.

### Description of Embodiments

Hereinafter, the embodiments for carrying out the present invention are described in detail. However, the present invention is not limited by the embodiments given below.

### (Terminology and the like)

The term "one or more" as used herein means the number of 1 or 2 or larger. When the term "one or more" is used in the context associated with the number of substituents of a group, the term means a number from 1 to the maximum number of substituents acceptable by the group. Specific examples of the term "one or more" include 1, 2, 3, 4, 5, 6, 7, 8, 9, 10, and/or larger numbers.

As used herein, the term "to" that indicates a numerical range includes values of both ends thereof. For example, "A to B" means the numerical range of A or more and B or less.

The term "about" as used herein, when used in combination with a numeric value, means the range of the value of +10% and -10% of the numeric value.

The term "and/or" as used herein is meant to include every combination of the terms "and" and "or" appropriately combined. Specifically, for example, the term "A, B, and/or C" includes the following 7 variations:
(i) A, (ii) B, (iii) C, (iv) A and B, (v) A and C, (vi) B and C, and (vii) A, B, and C.

The term "room temperature" as used herein means a temperature of about 20°C to about 25°C.

The term "N-carboxyanhydride (NCA)" as used herein refers to a compound in which an amino acid forms a cyclic structure via a carbonyl group (C=O). NCA can be synthesized, for example, by reacting an amino acid with thionyl chloride in the presence of a base.

NCA may be a compound represented by formula (1) (hereinafter also referred to as "compound 1"): 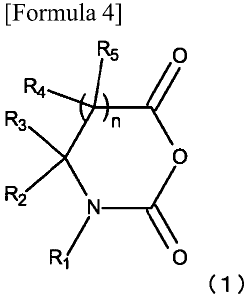
wherein n represents 0 or 1;
   (i) R₁ is hydrogen or C₁-C₈ alkyl, or
   (ii) R₁ and R₂, together with an atom to which they are bonded, form a 4- to 7-membered saturated heterocycle;
in the case of (i), R₂, R₃, R₄ and R₅ are each independently selected from the group consisting of hydrogen, C₁-C₆ alkyl, C₃-C₈ cycloalkyl, C₆-C₁₀ aryl C₁-C₆ alkyl, and 5- to 10-membered heteroaryl C₁-C₆ alkyl, each of which may be substituted by one or more groups independently selected from the group consisting of halogen, hydroxy, C₁-C₃ alkyl, halo C₁-C₆ alkyl, and C₁-C₆ alkoxycarbonyl, or R₂ and R₃, or R₄ and R₅, together with an atom to which they are each bonded, may form a 3- to 8-membered alicyclic ring or a 4- to 7-membered saturated heterocycle;
in the case of (ii), R₃, R₄, and R₅ are each independently selected from the group consisting of hydrogen, C₁-C₆ alkyl, C₃-C₈ cycloalkyl, C₆-C₁₀ aryl C₁-C₆ alkyl, and 5- to 10-membered heteroaryl C₁-C₆ alkyl, each of which may be substituted by one or more groups independently selected from the group consisting of halogen, hydroxy, C₁-C₃ alkyl, halo C₁-C₆ alkyl, and C₁-C₆ alkoxycarbonyl, or R₄ and R₅, together with an atom to which they are each bonded, may form a 3- to 8-membered alicyclic ring or a 4- to 7-membered saturated heterocycle.

In compound 1, n may be 0 or 1, preferably n is 0.

When n is 0, compound 1 is represented by the following formula (3):

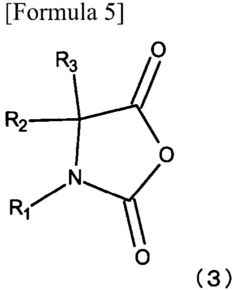

In compound 1, (i) R₁ may be hydrogen or C₁-C₈ alkyl, or (ii) R₁ and R₂, together with an atom to which they are bonded, may form a 4- to 7-membered saturated heterocycle. In compound 1, (i) R₁ is preferably hydrogen or C₁-C₈ alkyl.

In compound 1, as a preferred form of R₁, R₁ is preferably C₁-C₈ alkyl, more preferably C₁-C₃ alkyl, further preferably methyl or ethyl, and particularly preferably methyl.

In compound 1, as a preferred form of R₂, R₃, R₄ and R₅, R₂, R₃, R₄ and R₅ are each independently selected from the group consisting of hydrogen, C₁-C₆ alkyl, and C₆-C₁₀ aryl C₁-C₆ alkyl, each of which may be substituted by one or more groups independently selected from the group consisting of hydroxy, C₁-C₃ alkyl, halo C₁-C₆ alkyl, and C₁-C₆ alkoxycarbonyl.

In compound 1, as a preferred form of R₂, R₃, R₄ and R₅, R₂, R₃, R₄ and R₅ are each independently selected from the group consisting of hydrogen, C₁-C₆ alkyl, and C₆-C₁₀ aryl C₁-C₆ alkyl.

In compound 1, as a preferred form of compound 1, n is 0; R₁ is hydrogen or C₁-C₈ alkyl; R₂ and R₃ are each independently selected from the group consisting of hydrogen, C₁-C₆ alkyl, and C₆-C₁₀ aryl C₁-C₆ alkyl, each of which may be substituted by one or more groups independently selected from the group consisting of hydroxy, C₁-C₃ alkyl, halo C₁-C₆ alkyl, and C₁-C₆ alkoxycarbonyl.

In compound 1, as a more preferred form of compound 1, n is 0; R₁ is hydrogen or C₁-C₈ alkyl; R₂ and R₃ are each independently selected from the group consisting of hydrogen, C₁-C₆ alkyl, and C₆-C₁₀ aryl C₁-C₆ alkyl.

In compound 1, as a further preferred form of compound 1, n is 0, R₁ is methyl, R₂ is hydrogen, and R₃ is selected from the group consisting of methyl, ethyl, and benzyl.

The term "amino group-containing compound" as used herein means a compound having at least one amino group.

The amino group-containing compound may be selected from the group consisting of a peptide, an amino acid, an amino acid amide, and an amine. It is preferable that the amino group of the amino group-containing compound is a primary amino group or a secondary amino group.

The amino group-containing compound may be a compound selected from the group consisting of a peptide, an amino acid, and an amino acid amide, and in the compound selected from the group consisting of a peptide, an amino acid, and an amino acid amide, an amino group at the N-terminus is an amino group involved in a reaction of the step A. The reaction of the step A refers to a reaction of an N-carboxyanhydride(NCA) with an amino group of the amino group-containing compound.

The compound selected from the group consisting of a peptide, an amino acid, and an amino acid amide may be any of the following (a) to (c):
(a) a compound in which the amino group reacting with the NCA is a primary amino group;
(b) a compound in which the amino group reacting with the NCA is a secondary amino group, and a carbon atom to which the amino group reacting with the NCA is directly bonded is not substituted, wherein the carbon atom is a carbon atom in a main chain of an amino acid residue;
(c) a compound in which the amino group reacting with the NCA is a secondary amino group, and a 4- to 7-membered saturated heterocycle containing a nitrogen atom of the secondary amino group and a carbon atom to which the amino group reacting with the NCA is directly bonded is formed, wherein the carbon atom is a carbon atom in a main chain of an amino acid residue.

The amino group-containing compound may be an amino acid.

The amino group-containing compound may be an amino acid amide.

The amino group-containing compound may be a peptide, preferably a peptide composed of 2 to 20 amino acid residues.

The amino group-containing compound may be an amine, and is preferably selected from the group consisting of a C₁-C₈ alkylamine, a diC₁-C₈ alkylamine, and a compound containing a 4- to 7-membered saturated heterocycle containing a nitrogen atom of a secondary amino group, more preferably selected from the group consisting of methylamine, ethylamine, dimethylamine, diethylamine, morpholine, piperidine, pyrrolidine, and further preferably selected from the group consisting of dimethylamine, diethylamine, morpholine, piperidine, and pyrrolidine.

The term "peptide" as used herein means a compound wherein two or more amino acids are linked by an amide bond. Peptides having an ester bond in a portion of the main chain, such as depsipeptide, are also included in the peptide herein. The number of amino acid residues contained in the peptide is preferably 2 to 30 residues, more preferably 2 to 20 residues, further preferably 2 to 18 residues, and most preferably 2 to 15 residues. The peptide may be linear, branched, or cyclic.

The term "amino acid" as used herein refers to a carboxylic acid having an amino group, and includes a natural amino acid and a non-natural amino acid. The term "amino acid" as used herein may mean an amino acid residue. The term "natural amino acids" as used herein refers to glycine (Gly), L-alanine (Ala), L-serine (Ser), L-threonine (Thr), L-valine (Val), L-leucine (Leu), L-isoleucine (Ile), L-phenylalanine (Phe), L-tyrosine (Tyr), L-tryptophan (Trp), L-histidine (His), L-glutamic acid (Glu), L-aspartic acid (Asp), L-glutamine (Gln), L-asparagine (Asn), L-cysteine (Cys), L-methionine (Met), L-lysine (Lys), L-arginine (Arg), and L-proline (Pro).

The term "non-natural amino acid" as used herein means an amino acid other than the natural amino acid. Examples of the non-natural amino acid include a β-amino acid, a γ-amino acid, an L-type amino acid, a D-type amino acid, an N-substituted amino acid, an α,α-disubstituted amino acid, and an amino acid having a side chain different from that of natural amino acids. As the amino acid herein, amino acids having any conformation are acceptable. The side chains of the amino acids are not particularly limited, and each side chain is freely selected from groups such as alkyl, alkenyl, alkynyl, aryl, heteroaryl, aralkyl, heteroaralkyl, cycloalkyl, or spiro-bonded cycloalkyl, and/or atoms such as a hydrogen atom. Each group and/or atom is further optionally substituted. In one non-limiting aspect, the amino acid as used herein may be a compound having a carboxy group and an amino group in the same molecule. The nitrogen atom of the amino group and any atom of the side chain of the amino acid may be taken together to form a 4- to 7-membered saturated heterocycle. Examples of such amino acids include proline, hydroxyproline, and azetidine-2-carboxylic acid. The term "amino acid residue" as used herein means a moiety that corresponds to a unit of an amino acid that constitutes a peptide or protein on a peptide or protein molecule. As used herein, the term "amino acid residue" is sometimes referred to simply as an "amino acid".

The "side chain of amino acid" as used herein, in the case of an α-amino acid, means a group and/or atom bonded to a carbon (α-carbon) to which an amino group and a carboxyl group are bonded. For example, the methyl group of Ala is a side chain of the amino acid. In the case of a β-amino acid, a group and/or atom attached to an α-carbon and/or a β-carbon can be a side chain of the amino acid, and in the case of a γ-amino acid, a group and/or atom attached to an α-carbon, a β-carbon, and/or a γ-carbon can be a side chain of the amino acid.

The term "main chain of an amino acid" or "main chain of an amino acid residue" as used herein means a chain portion formed by an amino group, α-carbon and a carboxyl group in the case of an α-amino acid, a chain portion formed by an amino group, β-carbon, α-carbon and a carboxyl group in the case of a β-amino acid, and a chain portion formed by an amino group, γ-carbon, β-carbon, α-carbon and a carboxyl group in the case of a γ-amino acid.

The amino group in the main chain of the amino acid may be unsubstituted (-NH₂) or substituted (i.e., -NHR). wherein R represents alkyl, alkenyl, alkynyl, aryl, heteroaryl, aralkyl or cycloalkyl optionally having a substituent, or a carbon chain bonded to the nitrogen atom and a carbon atom at position α may form a ring as in proline). As used herein, such an amino acid in which an amino group in the main chain is substituted may be referred to as "N-substituted amino acid". Examples of the "N-substituted amino acid" as used herein include preferably, but are not limited to, N-alkylamino acid, N-ethylamino acid, N-C₁-C₆ alkylamino acid, N-C₁-C₄ alkylamino acid, N-methylamino acid, N-C₂-C₆ alkenylamino acid, N-allylamino acid, N-C₇-C₁₄ aralkylamino acid, N-benzylamino acid, and N-phenethylamino acid.

In some embodiments, the "amino acid" as used herein is isotopically labeled by replacing one or more atoms therein with an atom having the same atomic number (proton number) and a different mass number (total number of protons and neutrons) at an abundance ratio different from the natural abundance ratio. Such an isotopically-labeled (e.g., radiolabeled) amino acid is included in the "amino acid" herein. Examples of the isotope included in the "amino acid" as used herein include a hydrogen atom, a carbon atom, a nitrogen atom, an oxygen atom, a phosphorus atom, a sulfur atom, a fluorine atom, and a chlorine atom, and they include, for example, ²H, ³H; ¹³C, ¹⁴C; ¹⁵N; ¹⁷O, ¹⁸O; ³²P; ³⁵S; ¹⁸F; ³⁶Cl; and the like, respectively.

The term "amino acid amide" as used herein means a compound in which at least one carboxyl group of a natural amino acid or a non-natural amino acid has been converted to an amide group.

The term "Lewis acid" as used herein means an atom, molecule, or ion having an empty electron orbit that can accept at least one electron pair. The Lewis acid is not particularly limited as long as it is a compound that can accept an electron pair, for example, it may be an organic compound or an inorganic compound. Examples of the organic compound include an ammonium ion containing a carbon and an organic acid (e.g., carboxylic acid). Examples of the inorganic compound include a metal ion and a non-metal ion, and it may contain either one or both. Examples of the metal ion include a typical metal ion and a transition metal ion, and it may contain either one or both.

The Lewis acid herein is preferably a metal salt.

The metal ion of the metal salt is preferably at least one selected from the group consisting of a calcium ion, a zinc ion, a lanthanum ion, a terbium ion, a holmium ion, a thulium ion, a scandium ion, a bismuth ion, an iron ion, a barium ion, a cerium ion, a neodymium ion, a samarium ion, an europium ion, a gadolinium ion, an erbium ion, a lutetium ion, an ytterbium ion, an indium ion, a vanadium ion, a hafnium ion, and a strontium ion, more preferably at least one selected from the group consisting of a calcium ion, a zinc ion, a lanthanum ion, a terbium ion, a holmium ion, a thulium ion, a scandium ion, a bismuth ion, an iron ion, a barium ion, a cerium ion, a neodymium ion, a samarium ion, an europium ion, a gadolinium ion, an erbium ion, and a lutetium ion, and further preferably a calcium ion or a lanthanum.

The anion of the metal salt is preferably at least one selected from the group consisting of a trifluoromethanesulfonate ion, an iodide ion, a bromide ion, a chloride ion, and a nitrate ion, and more preferably at least one selected from the group consisting of a trifluoromethanesulfonate ion and an iodide ion.

The metal salt is preferably at least one selected from the group consisting of calcium trifluoromethanesulfonate, calcium iodide, zinc trifluoromethanesulfonate, lanthanum(III) trifluoromethanesulfonate, terbium(III) trifluoromethanesulfonate, holmium(III) trifluoromethanesulfonate, thulium(III) trifluoromethanesulfonate, scandium(III) trifluoromethanesulfonate, bismuth(III) trifluoromethanesulfonate, iron(II) trifluoromethanesulfonate, barium(II) trifluoromethanesulfonate, lanthanum(III) chloride bis(lithium chloride) complex, cerium(III) trifluoromethanesulfonate, neodymium(III) trifluoromethanesulfonate, samarium(III) trifluoromethanesulfonate, europium(III) trifluoromethanesulfonate, gadolinium(III) trifluoromethanesulfonate, erbium(III) trifluoromethanesulfonate, lutetium(III) trifluoromethanesulfonate, ytterbium(III) trifluoromethanesulfonate, zinc chloride, indium(III) bromide, vanadium(III) chloride, hafnium(IV) trifluoromethanesulfonate, lanthanum(III) nitrate hexahydrate, and strontium(II) trifluoromethanesulfonate, more preferably at least one selected from the group consisting of calcium trifluoromethanesulfonate, calcium iodide, zinc trifluoromethanesulfonate, lanthanum(III) trifluoromethanesulfonate, terbium(III) trifluoromethanesulfonate, holmium(III) trifluoromethanesulfonate, thulium(III) trifluoromethanesulfonate, scandium(III) trifluoromethanesulfonate, bismuth(III) trifluoromethanesulfonate, iron(II) trifluoromethanesulfonate, barium(II) trifluoromethanesulfonate, lanthanum(III) chloride bis(lithium chloride) complex, cerium(III) trifluoromethanesulfonate, neodymium(III) trifluoromethanesulfonate, samarium(III) trifluoromethanesulfonate, europium(III) trifluoromethanesulfonate, gadolinium(III) trifluoromethanesulfonate, erbium(III) trifluoromethanesulfonate, and lutetium(III) trifluoromethanesulfonate, further preferably at least one selected from the group consisting of calcium trifluoromethanesulfonate, calcium iodide, zinc trifluoromethanesulfonate, lanthanum(III) trifluoromethanesulfonate, terbium(III) trifluoromethanesulfonate, holmium(III) trifluoromethanesulfonate, and thulium(III) trifluoromethanesulfonate, and particularly preferably calcium iodide or lanthanum(III) trifluoromethanesulfonate.

The Lewis acid is preferably a hard Lewis acid or a borderlineLewis acid, and more preferably a hard Lewis acid, according to the HSAB theory. The HSAB theory (Hard and Soft Acids and Bases theory) is known to classify the compatibility of Lewis acids and Lewis bases using the expressions hard and soft. A "hard Lewis acid" is a cation that has a high charge density, is difficult to polarize, and is small in size. A "soft Lewis acid" is a cation that has a low charge density, is relatively easy to polarize, and is large in size. On the other hand, the "hard Lewis base" is a small base that has a large electronegativity and is difficult to polarize, and the "soft Lewis base" is a large base that has a small electronegativity and is easy to polarize. Borderline acids (borderline Lewis acids) and borderline bases (borderline Lewis bases) of these are also present. According to the HSAB theory, "hard Lewis acid" and "hard Lewis base", "soft Lewis acid" and "soft Lewis base" are easy to interact with each other, which is a rule of thumb. Examples of the hard Lewis acid and borderline Lewis acid are described in, for example, R. G. Pearson, J. Chem. Educ., 45, 581 (1968).

In addition, the value E^{≠}, which is a reference value for the hardness and softness of Lewis acid, is described in G. Klopman, J. Amer. Chem. Soc. 90, 223 (1968). The Lewis acid is preferably a Lewis acid having E^{≠} of 0.69 or more, and more preferably a Lewis acid having E^{≠} of 2.33 or more.

The term "optionally substituted" as used herein means that a certain group and/or atom may be substituted with an optional substituent and/or atom. That is, either of the state in which the certain group and atom are neither substituted by an optional substituent or optional atom, or the state in which the certain group and/or atom are substituted by an optional substituent and/or atom can be selected. An optional substituent and/or atom may be further added to the certain group and/or atom. That is, either of the state in which neither optional substituent nor optional atom may be added to the certain group and atom, or the state in which an optional substituent and/or atom is added to the certain group and/or atom can be selected. The optional substituent is not limited and may be, for example, freely selected from groups derived from atoms selected from the group consisting of a halogen atom, an oxygen atom, a sulfur atom, a nitrogen atom, a boron atom, a silicon atom, and a phosphorus atom. The certain atom is not also limited and may be, for example, freely selected from a halogen atom, an oxygen atom, a sulfur atom, a nitrogen atom, a boron atom, a silicon atom, and a phosphorus atom. Examples of the optional substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxyl, alkoxycarbonyl, and formyl.

The term "halogen" as used herein include fluorine, chlorine, bromine, and iodine.

The term "alkyl" as used herein is a linear or branched monovalent saturated hydrocarbon group derived from an aliphatic saturated hydrocarbon by removing any one hydrogen atom, the group having a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl is preferably alkyl having 1 to 20 carbon atoms (C₁-C₂₀; hereinafter, "Cₚ-C_{q}" means that the number of carbon atoms is p to q), more preferably C₁-C₁₅ alkyl, further preferably C₁-C₁₀ alkyl, and particularly preferably C₁-C₆ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, and n-hexyl. Further examples thereof include 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The term "cycloalkyl" as used herein is a saturated or partially saturated cyclic monovalent aliphatic hydrocarbon group and includes a monocyclic ring, a condensed ring, and a spiro ring. The cycloalkyl is preferably C₃-C₁₀ cycloalkyl, more preferably C₃-C₈ cycloalkyl, further preferably C₃-C₇ cycloalkyl, and particularly preferably C₃-C₆ cycloalkyl. Specific examples of the cycloalkyl include cyclopropyl, cyclobutyl, cyclopentyl, cyclohexyl, cycloheptyl, cyclooctyl, bicyclo[2.2.1]heptyl, and spiro[3.3]heptyl.

The term "aryl" as used herein is a monovalent aromatic hydrocarbon ring. The aryl is preferably C₆-C₁₀ aryl, more preferably C₆ aryl, C₈ aryl and C₁₀ aryl, further preferably C₆ aryl and C₁₀ aryl, and particularly preferably C₆ aryl. Specific examples of the aryl include phenyl, 1-naphthyl, 2-naphthyl, tolyl, and xylyl.

The term "heteroaryl" as used herein is a monovalent aromatic heterocyclic group that contains at least one heteroatom in addition to the carbon atom and is composed of a monocyclic or condensed ring that exhibits aromaticity. The heteroaryl composed of a monocycle is referred to herein as a monocyclic heteroaryl. The number of atoms constituting the ring of heteroaryl is, for example, 5 to 14 (5- to 14-membered heteroaryl), preferably 5 to 13 (5- to 13-membered heteroaryl), more preferably 5 to 10 (5- to 10-membered heteroaryl), particularly preferably 5 to 7 (5- to 7-membered heteroaryl). Specific examples of the heteroaryl include 5-membered heteroaryl such as furyl, thienyl, pyrrolyl, imidazolyl, pyrazolyl, thiazolyl, oxazolyl, triazolyl, or tetrazolyl; 6-membered heteroaryl such as pyridyl, pyrimidyl, pyridazinyl, pyrazinyl, or triazinyl; 8-membered heteroaryl such as benzofuranyl, benzothienyl, benzothiazolyl, benzimidazolyl, benzotriazolyl, indolyl, indazolyl, or pyrazolopyridyl; and 10-membered heteroaryl such as quinolyl, isoquinolyl, cinnolinyl, quinazolinyl, or quinoxalinyl.

The term "arylalkyl (aralkyl)" as used herein means a group in which at least one hydrogen atom of the "alkyl" defined above is replaced with the "aryl" as defined above. As the arylalkyl, C₇-C₁₄ arylalkyl is preferred, and C₇-C₁₀ arylalkyl is more preferred. Specific examples of the arylalkyl include benzyl, phenethyl, and 3-phenylpropyl.

The term "heteroarylalkyl" as used herein means a group in which at least one hydrogen atom of the "alkyl" as defined above is replaced with the "heteroaryl" as defined above. As the heteroarylalkyl, 5- to 10-membered heteroaryl-C₁-C₆ alkyl is preferred, and 5- to 7-membered heteroaryl-C₁-C₃ alkyl is more preferred. Specific examples of the heteroarylalkyl include imidazolylmethyl and indolemethyl.

The term "alicyclic ring" as used herein means a nonaromatic hydrocarbon ring. The alicyclic ring may have an unsaturated bond in the ring and may be a polycyclic ring having two or more rings. A carbon atom constituting the ring may form carbonyl through oxidation. The alicyclic ring is preferably a 3- to 8-membered alicyclic ring. Specific examples of the alicyclic ring include a cyclopropane ring, a cyclobutane ring, a cyclopentane ring, a cyclohexane ring, a cycloheptane ring, and a cyclooctane ring.

The term "saturated heterocycle" as used herein means a nonaromatic heterocycle containing at least one heteroatom in addition to a carbon atom, without containing a double bond and/or a triple bond in the ring. The saturated heterocycle may be a monocyclic ring or may form a condensed ring with another ring, for example, an aromatic ring such as a benzene ring. Preferred examples of the saturated heterocycle include a 4- to 10-membered saturated heterocyclic ring. Specific examples of the saturated heterocycle include an azetidine ring, an oxoazetidine ring, an oxetane ring, a tetrahydrofuran ring, a tetrahydropyran ring, a morpholine ring, a thiomorpholine ring, a pyrrolidine ring, a 2-oxopyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an indoline ring, an azepane ring, and a dioxepane ring.

The term "alkoxy" as used herein means an oxy group to which the "alkyl" as defined above is bonded. Preferred examples of the alkoxy include C₁-C₆ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The term "alkoxycarbonyl" as used herein means a carbonyl group to which the "alkoxy" as defined above is bonded. Preferred examples of the alkoxycarbonyl include C₁-C₆ alkoxycarbonyl. Specific examples of the alkoxycarbonyl include t-butoxycarbonyl.

The term "haloalkyl" as used herein means a group in which one or more hydrogen of the "alkyl" defined above are replaced with halogen. The haloalkyl is preferably halo C₁-C₆ alkyl, and more preferably halo C₁-C₃ alkyl. Specific examples of the haloalkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

The term "amino" as used herein means -NRR', wherein R and R' are each independently selected from hydrogen, alkyl, alkenyl, alkynyl, cycloalkyl, heterocyclyl, aryl, and heteroaryl, or R and R' form a ring together with the nitrogen atom to which they are bonded. Preferred examples of the amino include -NH₂, mono-C₁-C₆ alkylamino, di-C₁-C₆ alkylamino, and 4- to 7-membered cyclic amino.

If, in the production method described herein, a defined group undergoes undesired chemical conversion under conditions of the production method, the compounds can be produced, for example, by means such as protection or deprotection of the functional group. Here, for operations of selection and desorption of a protecting group, for example, methods described in "Greene's, "Protective Groups in Organic Synthesis" (5th edition, John Wiley & Sons 2014)" can be mentioned and may be appropriately used according to reaction conditions. It is also possible to change the order of reaction steps, such as steps of introducing a substituent, if necessary.

The compound described herein can be a salt thereof or a solvate thereof. Examples of the salt of the compound include hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salt and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the compound into contact with an acid or a base. The solvate of the compound is one in which the compound and a solvent together form a molecular aggregate, and is not particularly limited as long as it is a solvate formed by a solvent. Examples of the solvate include a hydrate, an alcohol solvate (such as ethanol solvate, methanol solvate, 1-propanol solvate, or 2-propanol solvate), and not only solvates formed with a single solvent such as dimethyl sulfoxide, but also solvates formed with a plurality of solvents per one molecule of the compound, or solvates formed with a plurality of types of solvents per one molecule of the compound. When the solvent is water, the solvate is called a hydrate. Examples of the solvate of the compound according to the present invention is preferably a hydrate, and specific examples of the hydrate include mono- to decahydrate, preferably mono- to pentahydrate, more preferably mono- to trihydrate.

The term "amide group-containing compound" as used herein means a compound having at least one amide group. Preferred examples of the amide group-containing compound include a peptide, more preferably a peptide containing 2 to 20 amino acid residues, further preferably a peptide containing 2 to 5 amino acid residues. As the amide group-containing compound, a peptide containing an N-substituted amino acid is preferred. The term "amide" as used herein means -NR-C(=O)-, wherein R is selected from hydrogen or C₁-C₈ alkyl, or R forms a 4- to 7-membered saturated heterocycle containing the nitrogen atom to which R is bonded. The 4- to 7-membered saturated heterocycle is a ring that is free of a carbonyl group in the main chain of amino acid residues. When R forms a 4- to 7-membered saturated heterocycle containing the nitrogen atom to which R is bonded, examples of the structure include the following:

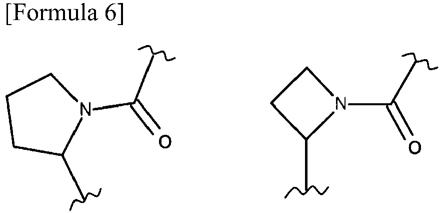

The term "over-elongation product" as used herein refers to an amide group-containing compound in which two or more NCA-derived amino acid residues are consecutively introduced into the amino group-containing compound. Preferred examples of the over-elongation product include an amide group-containing compound in which two or more and ten or less NCA-derived amino acid residues are consecutively introduced, more preferably a compound in which two or more and four or less NCA-derived amino acid residues are consecutively introduced, and further preferably an amide group-containing compound where NCAs in which two NCA-derived amino acid residues are consecutively introduced are consecutively reacted.

Examples and meanings of the abbreviations as used herein are listed below.
MeTHF: 2-methyltetrahydrofuran
THF: tetrahydrofuran
MeCN: acetonitrile
Ca(OTf)₂: calcium trifluoromethanesulfonate
La(OTf)₃: lanthanum(III) trifluoromethanesulfonate
CaI₂: calcium iodide
Zn(OTf)₂: zinc trifluoromethanesulfonate
Tb(OTf)₃: terbium(III) trifluoromethanesulfonate
Ho(OTf)₃: holmium(III) trifluoromethanesulfonate
Tm(OTf)₃: thulium(III) trifluoromethanesulfonate
Sc(OTf)₃: scandium(III) trifluoromethanesulfonate
Bi(OTf)₃: bismuth(III) trifluoromethanesulfonate
Fe(OTf)₂: iron(II) trifluoromethanesulfonate
Ba(OTf)₂: barium(III) trifluoromethanesulfonate
LaCl₃·2LiCl: lanthanum(III) chloride bis(lithium chloride) complex
Ce(OTf)₃: cerium(III) trifluoromethanesulfonate
Nd(OTf)₃: neodymium(III) trifluoromethanesulfonate
Sm(OTf)₃: samarium(III) trifluoromethanesulfonate
Eu(OTf)₃: europium(III) trifluoromethanesulfonate
Gd(OTf)₃: gadolinium(III) trifluoromethanesulfonate
Er(OTf)₃: erbium(III) trifluoromethanesulfonate
Lu(OTf)₃: lutetium(III) trifluoromethanesulfonate
Yb(OTf)₃: ytterbium(III) trifluoromethanesulfonate
ZnCl₂: zinc(II) chloride
InBr₃: indium(III) bromide
VCl₃: vanadium(III) chloride
Hf(OTf)₄: hafnium(III) trifluoromethanesulfonate
La(NO₃)₃·6H₂O: lanthanum(III) nitrate hexahydrate
DIPEA: N,N-diisopropylethylamine
NMM: N-methylmorpholine
K₂CO₃: potassium carbonate
KHCO₃: potassium hydrogen carbonate
CaO: calcium oxide
MgO: magnesium oxide
Cs₂CO₃: cesium carbonate
CsHCO₃: cesium hydrogen carbonate
K₃PO₄: potassium phosphate
K₂HPO₄: dipotassium hydrogen phosphate
NaHCO₃: sodium hydrogen carbonate
HATU: O-(7-azabenzotriazol-1-yl)-N,N,N',N'-tetramethyluronium hexafluorophosphate
tBu: tert-butyl
Bn: benzil
Boc: tert-butoxycarbonyl
Cbz: benzyloxycarbonyl
MS3A: molecular sieve 3A
T3P: propylphosphonic anhydride
CDI: carbonyl diimidazole
TfOH: trifluoromethanesulfonic acid
MsOH: methanesulfonic acid

### (Production method)

The method for producing an amide group-containing compound of the present embodiments includes step A of reacting an N-carboxyanhydride (NCA) with an amino group of an amino group-containing compound in a first solvent in the presence of a Lewis acid.

The amide group-containing compound obtained by the production method of the amide group-containing compound of the present embodiments has an amide group formed by the reaction between NCA and an amino group of an amino group-containing compound, in which a carbon atom of a carbonyl group derived from an amino acid in the NCA and a nitrogen atom of the amino group of the amino group-containing compound is bonded. According to the production method of the present embodiments, as shown in the following chemical formula, the reaction of the NCA with the amino group of the amino group-containing compound generates a carbamic acid intermediate, which is then decarboxylated to afford the amide group-containing compound. Without limiting by a particular theory, it is presumed that, in the production method of the present embodiments, the action of the Lewis acid suppresses the generation of over-elongation products.

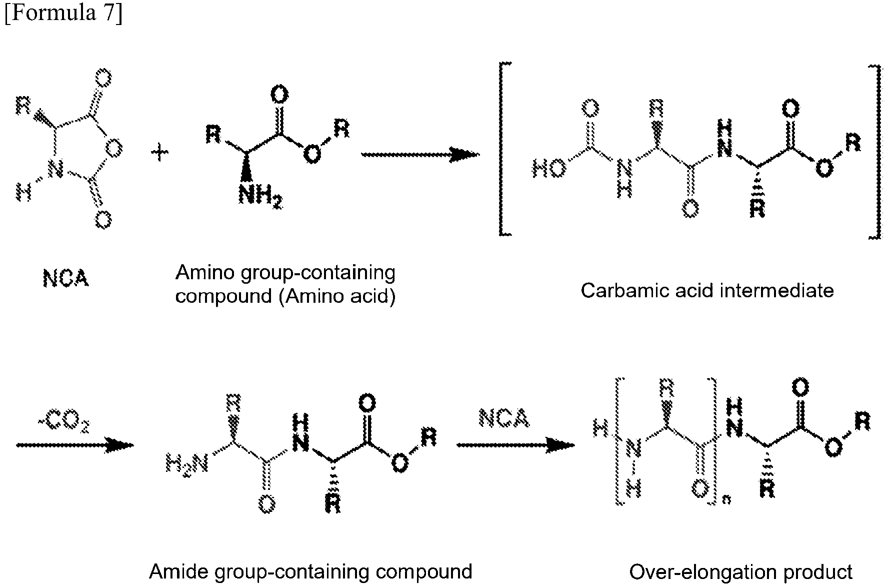

Furthermore, according to the production method of the present embodiments, an amide group-containing compound can be obtained in a high yield. Furthermore, according to the production method of the present embodiments, it is possible to control the reaction simply by adding a Lewis acid without requiring special reaction conditions, thus it is excellent in practicality.

In the step A, the liquid temperature of the first solvent may be -20°C or higher, -10°C or higher, 0°C or higher, 4°C or higher, 8°C or higher, 12°C or higher, 16°C or higher, or 20°C or higher, and may be 100°C or lower, 80°C or lower, 50°C or lower, 40°C or lower, 35°C or lower, 30°C or lower, or 25°C or lower. In the step A, the liquid temperature of the first solvent may be -20 to 100°C, 4 to 30°C, 20 to 40°C or 20 to 30°C. In the step A, the liquid temperature of the first solvent is preferably -20 to 100°C, more preferably 0 to 40°C, and particularly preferably 20 to 30°C.

In the step A, the time for reacting the N-carboxyamino acid anhydride (NCA) with the amino group of the amino group-containing compound in the first solvent in the presence of the Lewis acid described above may be 0.5 hours or more, 1 hour or more, 1.5 hours or more, or 2 hours or more, and may be 72 hours or less, 48 hours or less, 36 hours or less, 24 hours or less, 12 hours or less, 6 hours or less, or 5 hours or less.

Examples of the first solvent include a cyclic ether-based solvent, an aromatic hydrocarbon-based solvent, an ester-based solvent, a nitrile-based solvent, and an amide-based solvent. Specific examples thereof include tetrahydrofuran, 2-methyltetrahydrofuran, 4-methyltetrahydropyran, toluene, isopropyl acetate, acetonitrile, and 1,3-dimethyl-2-imidazolidinone. The first solvent is preferably a solvent containing at least one selected from the group consisting of 2-methyltetrahydrofuran, toluene, tetrahydrofuran, 4-methyltetrahydropyran, isopropyl acetate, acetonitrile, and 1,3-dimethyl-2-imidazolidinone, more preferably a solvent containing at least one selected from the group consisting of 2-methyltetrahydrofuran, toluene, tetrahydrofuran, 4-methyltetrahydropyran, and isopropyl acetate, and further preferably a solvent containing at least one selected from the group consisting of 2-methyltetrahydrofuran and toluene.

In the step A, the Lewis acid can be used preferably at 0.1 molar equivalents to 5.0 molar equivalents, more preferably 0.25 molar equivalents to 2.0 molar equivalents, further preferably 0.25 molar equivalents to 1.0 molar equivalent, and particularly preferably 0.25 molar equivalents to 0.5 molar equivalents with respect to the amino group-containing compound.

In the step A, the NCA can be used preferably at 0.8 molar equivalents to 5.0 molar equivalents, more preferably 1.0 molar equivalent to 2.0 molar equivalents, and further preferably 1.1 molar equivalents to 1.5 molar equivalents with respect to the amino group-containing compound.

The step A can be carried out in the absence of a base and/or a desiccant. In addition, a nucleophilic reagent such as alcohol is not necessarily added to the reaction mixture after the step A.

The NCA used in the step A can be one produced using a conventionally known method, and may be one, for example, produced by a method including step B of contacting an amino acid protected with a Boc group with a first activator in a second solvent to produce the NCA, or a method including step B of contacting an amino acid with a second activator in a second solvent to produce the NCA.

The production method of the present invention may include repeating the step A two or more times, or three or more times.

An aspect of the present embodiments provides a production method in which the peak area of the over-elongation product is reduced to 1% or less with respect to the sum of peak areas of an amide group-containing compound into which one amino acid residue from NCA is introduced and the over-elongation product as determined by UVArea% at 210 nm by HPLC analysis. In an embodiment, the value above is 1% or less, 0.5% or less, or undetectable.

An aspect of the present embodiments provides a suppressor containing a Lewis acid, which suppresses the generation of an over-elongation product in the production of an amide group-containing compound using NCA. The foregoing definitions herein are used for the definitions of each term in the present embodiments.

An aspect of the present embodiments relates to a production method of an amide group-containing compound, which may include, prior to the step A, step B of contacting an amino acid protected with a Boc group with a first activator in a second solvent to produce the NCA.

The amino acid protected with a Boc group may be, for example, a compound represented by the following formula (2) (hereinafter also referred to as "compound 2"): 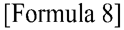 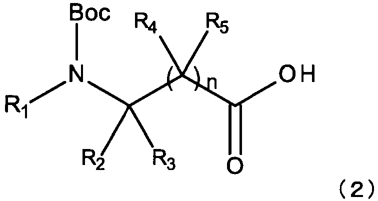 wherein n, R₁, R₂, R₃, R₄ and R₅ has the same meaning as n, R₁, R₂, R₃, R₄ and R₅ in formula (1), respectively.

When n is 0, compound 2 is represented by the following formula (4):

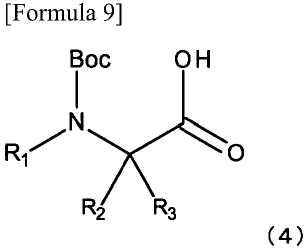

In the step B, the liquid temperature of the second solvent may be -20°C or higher, -10°C or higher, 0°C or higher, 4°C or higher, 8°C or higher, 12°C or higher, 16°C or higher, or 20°C or higher, and may be 100°C or lower, 80°C or lower, 50°C or lower, 40°C or lower, 35°C or lower, 30°C or lower, or 25°C or lower. In the step B, the liquid temperature of the second solvent may be -20 to 100°C, 4 to 30°C, 20 to 40°C or 20 to 30°C. In the step B, the liquid temperature of the second solvent is preferably -20 to 100°C, more preferably 20 to 40°C, and further preferably 20 to 30°C.

In the step B, the time for contacting the amino acid protected with a Boc group with the first activator in the second solvent described above may be 0.5 hours or more, 1 hour or more, 1.5 hours or more, or 2 hours or more, and may be 72 hours or less, 48 hours or less, 36 hours or less, 24 hours or less, 12 hours or less, 6 hours or less, or 5 hours or less. In the step B, the time for contacting the amino acid protected with a Boc group with the first activator in the second solvent described above may be 0.5 to 72 hours, 0.5 to 48 hours, 0.5 to 5 hours, 2 to 48 hours, or 2 to 5 hours. In the step B, the time for contacting the amino acid protected with a Boc group with the first activator in the second solvent described above is preferably 0.5 to 72 hours, more preferably 0.5 to 48 hours, and further preferably 2 to 5 hours.

In an aspect of the present embodiments, a reaction mixture containing the NCA obtained in the step B may be used directly in the step A.

In an aspect of the present embodiments, the method may include, after the step B, step C of removing at least a portion of the second solvent and mixing with a third solvent, and the resulting solution containing the NCA may be used directly in the step A.

In an aspect of the present embodiments, the second solvent may be removed from a reaction mixture obtained in the step B, and the resulting mixture containing the NCA may be used without purification in the step A.

Examples of the first activator that can be used include phosphorus pentachloride, phosphorus oxychloride, phosphorus trichloride, propylphosphonic anhydride, oxalyl chloride, thionyl chloride, triphosgene, and dichloromethyl methyl ether. The first activator is preferably at least one selected from the group consisting of propyl phosphonic anhydride, thionyl chloride, oxalyl chloride, phosphorus trichloride, triphosgene and dichloromethyl methyl ether, more preferably at least one selected from the group consisting of propylphosphonic anhydride, thionyl chloride, oxalyl chloride and phosphorus trichloride, and further preferably thionyl chloride or propylphosphonic anhydride.

Examples of the second solvent include a cyclic ether-based solvent, an aromatic hydrocarbon-based solvent, an ester-based solvent, and a nitrile-based solvent. Specific examples thereof include tetrahydrofuran, 2-methyltetrahydrofuran, isopropyl acetate, ethyl acetate, toluene, and acetonitrile. The second solvent is preferably a solvent containing at least one selected from the group consisting of tetrahydrofuran, 2-methyltetrahydrofuran, isopropyl acetate, ethyl acetate, toluene, and acetonitrile, more preferably a solvent containing at least one selected from the group consisting of tetrahydrofuran, 2-methyltetrahydrofuran, isopropyl acetate, and ethyl acetate, and further preferably tetrahydrofuran or ethyl acetate.

Examples of the third solvent include a cyclic ether-based solvent, an aromatic hydrocarbon-based solvent, an ester-based solvent, and a nitrile-based solvent. Specific examples thereof include 2-methyltetrahydrofuran, isopropyl acetate, toluene, and acetonitrile. The third solvent is preferably a solvent containing at least one selected from the group consisting of 2-methyltetrahydrofuran, toluene, tetrahydrofuran, 4-methyltetrahydropyran, isopropyl acetate, acetonitrile, and 1,3-dimethyl-2-imidazolidinone, more preferably a solvent containing at least one selected from the group consisting of 2-methyltetrahydrofuran, toluene, tetrahydrofuran, 4-methyltetrahydropyran, and isopropyl acetate, and further preferably a solvent containing at least one selected from the group consisting of 2-methyltetrahydrofuran and toluene.

It is preferable that the third solvent includes the same solvent as the first solvent, and more preferably the third solvent is the same solvent as the first solvent.

In an aspect of the present embodiments, the step B may also be carried out in the presence of a base.

In an aspect of the present embodiments, a base may be added after the step B.

Examples of the base that can be used include DIPEA, cesium carbonate, sodium hydrogen carbonate, 1-methylpiperidine, 4-ethylmorpholine, N-methylmorpholine, 2,4,6-collidine, pyridine, potassium carbonate, potassium hydrogen carbonate, magnesium oxide, calcium oxide, tripotassium phosphate, dipotassium hydrogen phosphate, and cesium hydrogen carbonate. The base is preferably at least one selected from the group consisting of DIPEA, cesium carbonate, sodium hydrogen carbonate, 1-methylpiperidine, 4-ethylmorpholine, N-methylmorpholine, 2,4,6-collidine, pyridine, potassium carbonate, potassium hydrogen carbonate, magnesium oxide, calcium oxide, tripotassium phosphate, dipotassium hydrogen phosphate, and cesium hydrogen carbonate, more preferably at least one selected from the group consisting of DIPEA, cesium carbonate, sodium hydrogen carbonate, pyridine, potassium carbonate, calcium oxide, and cesium hydrogen carbonate, further preferably, at least one selected from the group consisting of cesium carbonate, pyridine, potassium carbonate, calcium oxide, and cesium hydrogen carbonate, and particularly preferably calcium oxide or pyridine.

An aspect of the present embodiments relates to a production method of an amide group-containing compound, which may include, prior to the step A, step B' of contacting an amino acid with a second activator in a second solvent to produce the NCA.

The amino acid may be, for example, a compound represented by the following formula (2') (hereinafter also referred to as "compound 2'"): 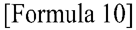 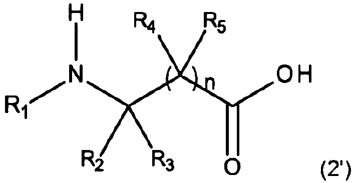 wherein n, R₁, R₂, R₃, R₄ and R₅ has the same meaning as n, R₁, R₂, R₃, R₄ and R₅ in formula (1), respectively.

When n is 0, compound 2' is represented by the following formula (4'):

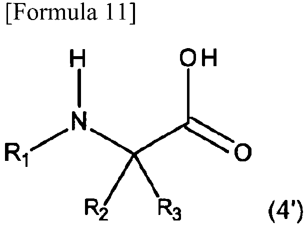

In the step B', the liquid temperature of the second solvent may be -20°C or higher, -10°C or higher, 0°C or higher, 4°C or higher, 8°C or higher, 12°C or higher, 16°C or higher, or 20°C or higher, and may be 100°C or lower, 80°C or lower, 50°C or lower, 40°C or lower, 35°C or lower, 30°C or lower, or 25°C or lower. In the step B', the liquid temperature of the second solvent may be -20 to 100°C, 4 to 30°C, 20 to 40°C or 20 to 30°C. In the step B', the liquid temperature of the second solvent is preferably -20 to 100°C, more preferably 20 to 40°C, and further preferably 20 to 30°C.

In the step B', the time for contacting an amino acid with a second activator in the second solvent described above may be 0.5 hours or more, 1 hour or more, 1.5 hours or more, or 2 hours or more, and may be 72 hours or less, 48 hours or less, 36 hours or less, 24 hours or less, 12 hours or less, 6 hours or less, or 5 hours or less. In the step B', the time for contacting an amino acid with a second activator in the second solvent described above may be 0.5 to 72 hours, 0.5 to 48 hours, 0.5 to 5 hours, 2 to 48 hours, or 2 to 5 hours. In the step B', the time for contacting an amino acid with a second activator in the second solvent described above is preferably 0.5 to 72 hours, more preferably 0.5 to 48 hours, and further preferably 2 to 5 hours.

In an aspect of the present embodiments, a reaction mixture containing the NCA obtained in the step B' may be used directly in the step A.

In an aspect of the present embodiments, the method may include, after the step B', step C of removing at least a portion of the second solvent and mixing with a third solvent, and the resulting solution containing the NCA may be used directly in the step A.

In an aspect of the present embodiments, the second solvent may be removed from a reaction mixture obtained in the step B', and the resulting mixture containing the NCA may be used without purification in the step A.

Examples of the second activator that can be used include a condensing agent having a carbonyl group. The second activator is preferably at least one selected from the group consisting of triphosgene, carbonyl imidazole, and di-tert-butyl dicarbonate.

Examples of the second solvent and the third solvent can include a solvent similar to the solvent described in the step B above as examples.

In an aspect of the present embodiments, the step B' may also be carried out in the presence of an acid.

Examples of the acid that can be used include trifluoromethanesulfonic acid, methanesulfonic acid, and imidazole hydrochloride.

### Examples

Hereinafter, the present disclosure will be described in more detail based on Examples, but the present disclosure is not limited to the following Examples. Except those as specifically described, starting substances, starting raw materials, solvents, and reagents were obtained from commercial suppliers, or synthesized using known methods.

In the following Examples, high performance liquid chromatography (HPLC) analysis was performed using any of the analysis conditions described below. Detection of each compound was performed using a photodiode array detector or mass spectrometer. LCMS analysis was performed using a QDa detector.

### HPLC analysis condition method 1

Apparatus: Waters ACQUITY UPLC H-Class
Column: Ascentis Express C18 (Supelco), 4.6 mm ID × 50 mm, 2.7 µm
Mobile phase: 0.05% TFA/water (A), 0.05% TFA/MeCN (B)
Elution method: B) 5% (0 min)→100% (6 min) → 100% (7 min) → 5% (7.1 min) → 5% (9 min)
Flow rate: 1 mL/min
Column temperature: 35°C
Detection wavelength: 210 nm (PDA)
Sample preparation: 5 µL of the reaction solution is diluted with 1 mL of MeCN.

### HPLC analysis condition method 2

Apparatus: Waters ACQUITY UPLC H-Class
Column: ACQUITY UPLC CSH fluoro-phenyl, 1.0 mm ID × 150 mm, 1.7 µm
Mobile phase: 0.05% TFA/water (A), 0.05% TFA/MeCN (B)
Elution method: B) 5% (0 min) → 80% (20 min) → 100% (20.1 min) → 100% (25 min) → 5% (25.1 min) → 5% (30 min)
Flow rate: 0.15 mL/min
Column temperature: 35°C
Detection wavelength: 210 nm (PDA)
Sample preparation: 5 µL of the reaction solution is diluted with 1 mL of MeCN.

### NMR analysis conditions

NMR measuring apparatus: JEOL
JNM-ECZ500R/S1
Measurement conditions (¹H-NMR): heavy solvent DMSO-d₆, temperature 22.0°C, pulse angle 45°, digital resolution 0.76 Hz, relaxation time 5 seconds, with spin, cumulative number 8 times

### Example 1:

### Synthesis of tert-butyl methyl-L-alanyl-L-phenylalaninate (compound 2A)

The reaction conditions for the synthesis of tert-butyl methyl-L-alanyl-L-phenylalaninate (compound 2A) were studied using tert-butyl L-phenylalaninate (compound 1A) and (S)-3,4-dimethyloxazolidine-2,5-dione (NCA1) as raw materials.

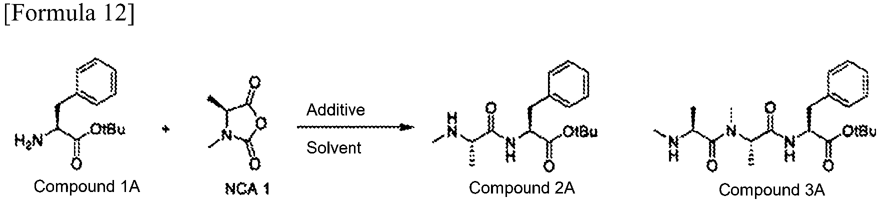

### Run 1

To a reaction vessel, tert-butyl L-phenylalaninate (50.1 mg), toluene (0.5 mL), and (S)-3,4-dimethyloxazolidine-2,5-dione (29.2 mg) were sequentially added and allowed to react at room temperature for 0.5 hours. The resulting reaction mixture was analyzed by HPLC analysis condition method 1.

### Run 2

To a reaction vessel, tert-butyl L-phenylalaninate (50.8 mg), toluene (0.5 mL), La(OTf)₃ (43.5 mg), and (S)-3,4-dimethyloxazolidine-2,5-dione (29.6 mg) were sequentially added. The mixture was stirred at room temperature for 6 hours, then MeCN (0.5 mL) was added, and the mixture was stirred for an additional 16 hours. The resulting reaction mixture was analyzed by HPLC analysis condition method 1.

### Run 3

To a reaction vessel, tert-butyl L-phenylalaninate (50.6 mg), toluene (0.5 mL), CaI₂ (32.6 mg), and (S)-3,4-dimethyloxazolidine-2,5-dione (29.5 mg) were sequentially added. The mixture was stirred at room temperature for 3 hours, then MeCN (0.5 mL) was added, and the mixture was allowed to react for an additional 22 hours. The resulting reaction mixture was analyzed by HPLC analysis condition method 1.

### Run 4

To a reaction vessel, tert-butyl L-phenylalaninate (49.9 mg), toluene (0.5 mL), Ca(OTf)₂ (37.2 mg) and (S)-3,4-dimethyloxazolidine-2,5-dione (30.0 mg) were sequentially added and allowed to react at room temperature for 3 hours. To the resulting reaction mixture, MeCN (0.5 mL) was added and homogenized, and the resulting mixture was analyzed by HPLC analysis condition method 1.

### Run 5

To a reaction vessel, tert-butyl L-phenylalaninate (50.1 mg), toluene (0.5 mL), Zn(OTf)₂ (27.4 mg) and (S)-3,4-dimethyloxazolidine-2,5-dione (32.5 mg) were sequentially added and allowed to react at room temperature for 8 hours. The resulting reaction mixture was analyzed by HPLC analysis condition method 1.

### Run 6

To a reaction vessel, tert-butyl L-phenylalaninate (51.8 mg), toluene (0.5 mL), Tb(OTf)₃ (45.7 mg) and (S)-3,4-dimethyloxazolidine-2,5-dione (29.7 mg) were sequentially added and allowed to react at room temperature for 6 hours. The resulting reaction mixture was analyzed by HPLC analysis condition method 1.

### Run 7

To a reaction vessel, tert-butyl L-phenylalaninate (50.9 mg), toluene (0.5 mL), Ho(OTf)₃ (47.1 mg) and (S)-3,4-dimethyloxazolidine-2,5-dione (29.7 mg) were sequentially added and allowed to react at room temperature for 6 hours. The resulting reaction mixture was analyzed by HPLC analysis condition method 1.

### Run 8

To a reaction vessel, tert-butyl L-phenylalaninate (49.8 mg), toluene (0.5 mL), Tm(OTf)₃ (47.8 mg) and (S)-3,4-dimethyloxazolidine-2,5-dione (29.5 mg) were sequentially added and allowed to react at room temperature for 6 hours. The resulting reaction mixture was analyzed by HPLC analysis condition method 1.

### Run 9 to Run 26

To a reaction vessel, tert-butyl L-phenylalaninate (50 mg), toluene (0.5 mL), an additive (0.25 to 0.5 eq.), and (S)-3,4-dimethyloxazolidine-2,5-dione (1.0 eq.) were sequentially added and allowed to react at room temperature for 0.5 to 22 hours. The resulting reaction mixture was analyzed by HPLC analysis condition method 1.

For the results of Runs 1 to 26, Tables 1 and 2 show the reaction conversion and selectivity calculated from the peak areas of compound 1A (raw material), compound 2A (target), and compound 3A (impurity) in each reaction mixture. The reaction conversion is calculated by the calculation formula "(compound 2A + compound 3A)/(compound 1A + compound 2A + compound 3A) × 100%". The selectivity (X/Y) was calculated by the calculation formulas "X = compound 2A/(compound 2A + compound 3A) × 100%, Y = compound 3A/(compound 2A + compound 3A) × 100%".

HPLC analysis data for each compound are shown below.
Compound 1A: retention time 2.4 min, LCMS m/z 222 [M+H]⁺
Compound 2A: retention time 2.6 min, LCMS m/z 307 [M+H]⁺
Compound 3A: retention time 2.8 min, LCMS m/z 392 [M+H]⁺

**[Table 1]**

| Run | NCA (equivalent) | Additive (equivalent) | Reaction conversion | Selectivity |
|---|---|---|---|---|
| 1 | 1.0 eq. | None | 95.6 | 94.1/5.9 |
| 2 | 1.0 eq. | La(OTf)₃ (0.33 eq.) | 92.2 | 99.7/0.3 |
| 3 | 1.0 eq. | CaI₂ (0.5 eq.) | 93.7 | 99.7/0.3 |
| 4 | 1.0 eq. | Ca(OTf)₂ (0.5 eq.) | 98.5 | 99.1/0.9 |
| 5 | 1.1 eq. | Zn(OTf)₂ (0.33 eq.) | 92.5 | 99.0/1.0 |
| 6 | 1.0 eq. | Tb(OTf)₃ (0.33 eq.) | 95.8 | 99.3/0.7 |
| 7 | 1.0 eq. | Ho(OTf)₃ (0.33 eq.) | 91.3 | 99.5/0.5 |
| 8 | 1.0 eq. | Tm(OTf)₃ (0.33 eq.) | 90.0 | 99.6/0.4 |

**[Table 2]**

| Run | Additive (equivalent) | Reaction time (h) | Reaction conversion | Selectivity |
|---|---|---|---|---|
| 1 | None | 0.5 | 95.6 | 94.1/5.9 |
| 9 | Sc(OTf)₃ (0.33 eq.) | 6 | 93.2 | 98.2/1.8 |
| 10 | Bi(OTf)₃ (0.33 eq.) | 6 | 91.3 | 98.1/1.9 |
| 11 | Fe(OTf)₂ (0.5 eq.) | 22 | 86.6 | 99.7/0.3 |
| 12 | Ba(OTf)₂ (0.5 eq.) | 3 | 93.5 | 98.5/1.5 |
| 13 | LaCl₃·2LiCl (0.33 eq.) | 22 | 87.0 | 99.5/0.5 |
| 14 | Ce(OTf)₃ (0.33 eq.) | 6 | 83.3 | 99.1/0.9 |
| 15 | Nd(OTf)₃ (0.33 eq.) | 22 | 84.7 | 99.7/0.3 |
| 16 | Sm(OTf)₃ (0.33 eq.) | 6 | 88.8 | 99.7/0.3 |
| 17 | Eu(OTf)₃ (0.33 eq.) | 22 | 83.9 | 99.7/0.3 |
| 18 | Gd(OTf)₃ (0.33 eq.) | 6 | 85.9 | 99.8/0.2 |
| 19 | Er(OTf)₃ (0.33 eq.) | 22 | 83.4 | 99.7/0.3 |
| 20 | Lu(OTf)₃ (0.33 eq.) | 6 | 94.4 | 98.9/1.1 |
| 21 | Yb(OTf)₃ (0.33 eq.) | 3 | 96.0 | 96.3/3.7 |
| 22 | ZnCl₂ (0.5 eq.) | 7 | 83.7 | 96.5/3.5 |
| 23 | InBr₃ (0.33 eq.) | 6 | 89.7 | 97.1/2.9 |
| 24 | VCl₃ (0.5 eq.) | 6 | 92.4 | 95.3/4.7 |
| 25 | Hf(OTf)₄ (0.25 eq.) | 3 | 96.9 | 95.5/4.5 |
| 26 | La(NO₃)₃·6H₂O (0.33 eq.) | 3 | 84.8 | 98.3/1.7 |

As is clear from Tables 1 and 2, a large amount of over-elongation product (compound 3A), which is an impurity, was generated when no additive was added (Run 1). In contrast, when the Lewis acid such as Ca(OTf)₂, CaI₂, Zn(OTf)₂, or La(OTf)₃ was added, the generation of the over-elongation product (compound 3A), which is an impurity, was reduced, and compound 2A (target) was selectively obtained (Runs 2 to 26).

### Example 2:

### Synthesis of tert-butyl methyl-L-phenylalanyl-L-phenylalaninate (compound 2B)

The reaction conditions for the synthesis of tert-butyl methyl-L-phenylalanyl-L-phenylalaninate (compound 2B) were studied using tert-butyl L-phenylalaninate (compound 1A) and (S)-4-benzyl-3-methyloxazolidine-2,5-dione (NCA2) as raw materials.

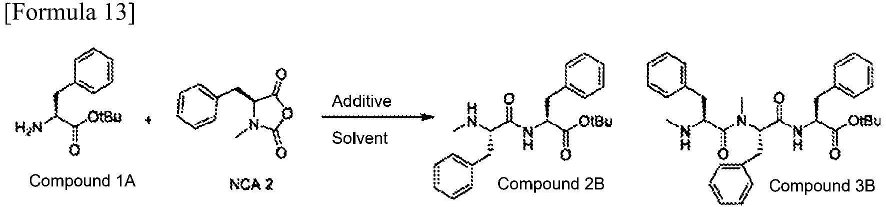

### Run 1

To a reaction vessel, tert-butyl L-phenylalaninate (50.4 mg), MeTHF (0.5 mL), and (S)-4-benzyl-3-methyloxazolidine-2,5-dione (50.9 mg) were sequentially added and allowed to react at room temperature for 22 hours. The resulting reaction mixture was analyzed by HPLC analysis condition method 1.

### Run 2

To a reaction vessel, tert-butyl L-phenylalaninate (50.9 mg), MeTHF (0.5 mL), La(OTf)₃ (43.8 mg), molecular sieve 3A (50 mg) and (S)-4-benzyl-3-methyloxazolidine-2,5-dione (51.1 mg) were sequentially added and allowed to react at room temperature for 22 hours. The resulting reaction mixture was analyzed by HPLC analysis condition method 1.

### Run 3

To a reaction vessel, tert-butyl L-phenylalaninate (50.1 mg), MeTHF (0.5 mL), CaI₂ (33.6 mg) and (S)-4-benzyl-3-methyloxazolidine-2,5-dione (50.9 mg) were sequentially added and allowed to react at room temperature for 22 hours. The resulting reaction mixture was analyzed by HPLC analysis condition method 1.

For the results of Runs 1 to 3, Table 3 shows the peak area ratios (%) of compound 1A (raw material), compound 2B (target), and compound 3B (impurity) in each reaction mixture.

HPLC analysis data for each compound are shown below.
Compound 2B: retention time 3.2 min, LCMS m/z 383 [M+H]⁺
Compound 3B: retention time 3.7 min, LCMS m/z 544 [M+H]⁺

**[Table 3]**

| Run | Additive (equivalent) | Compound 1A | Compound 2B | Compound 3B |
|---|---|---|---|---|
| 1 | None | <0.1 | 98.15 | 1.85 |
| 2 | La(OTf)₃ (0.33 eq.) | 0.32 | 99.37 | 0.31 |
| 3 | CaI₂ (0.5 eq.) | 0.16 | 99.64 | 0.20 |

As is clear from Table 3, a large amount of over-elongation product (compound 3B), which is an impurity, was generated when no additive was added (Run 1). In contrast, when the Lewis acid was added, the generation of the over-elongation product (compound 3B), which is an impurity, was reduced, and compound 2B (target) was selectively obtained (Runs 2 to 3).

### Example 3:

### Synthesis of tert-butyl butyl-glycyl-L-phenylalaninate (compound 2C)

The reaction conditions for the synthesis of tert-butyl butyl-glycyl-L-phenylalaninate (compound 2C) were studied using tert-butyl L-phenylalaninate (compound 1A) and 3-butyloxazolidine-2,5-dione (NCA3) as raw materials.

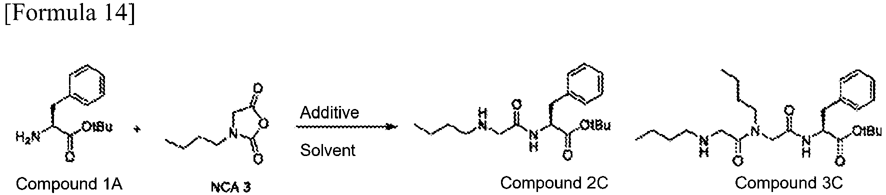

### Run 1

To a reaction vessel, tert-butyl L-phenylalaninate (50.2 mg), MeTHF (0.5 mL), and 3-butyloxazolidine-2,5-dione (36.3 mg) were sequentially added and allowed to react at room temperature for 0.5 hours. The resulting reaction mixture was analyzed by HPLC analysis condition method 1.

### Run 2

To a reaction vessel, tert-butyl L-phenylalaninate (49.9 mg), MeTHF (0.5 mL), La(OTf)₃ (44.9 mg) and 3-butyloxazolidine-2,5-dione (36.3 mg) were sequentially added and allowed to react at room temperature for 27 hours. The resulting reaction mixture was analyzed by HPLC analysis condition method 1.

For the results of Runs 1 to 2, Table 4 shows the peak area ratios (%) of compound 1A (raw material), compound 2C (target), and compound 3C (impurity) in each reaction mixture.

HPLC analysis data for each compound are shown below.
Compound 2C: retention time 2.7 min, LCMS m/z 335 [M+H]⁺
Compound 3C: retention time 3.7 min, LCMS m/z 448 [M+H]⁺

**[Table 4]**

| Run | Additive (equivalent) | Compound 1A | Compound 2C | Compound 3C |
|---|---|---|---|---|
| 1 | None | 16.12 | 74.32 | 9.56 |
| 2 | La(OTf)₃ (0.33 eq.) | 30.39 | 68.97 | 0.64 |

As is clear from Table 4, a large amount of over-elongation product (compound 3C), which is an impurity, was generated when no additive was added (Run 1). In contrast, when the Lewis acid was added, the generation of the over-elongation product (compound 3C), which is an impurity, was reduced, and compound 2C (target) was selectively obtained (Run 2).

### Example 4:

### Synthesis of tert-butyl (S)-4-(dimethylamino)-3-((S)-N-methyl-2-((S)-2-(methylamino)propanamide)-3-phenylpropanamide)-4-oxobutanoate (compound 2D)

The reaction conditions for the synthesis of tert-butyl (S)-4-(dimethylamino)-3-((S)-N-methyl-2-((S)-2-(methylamino)propanamide)-3-phenylpropanamide)-4-oxobutanoate (compound 2D) were studied using tert-butyl (S)-3-((S)-2-amino-N-methyl-3-phenylpropanamide)-4-(dimethylamino)-4-oxobutanoate (compound 1B) and (S)-3,4-dimethyloxazolidine-2,5-dione (NCA1) as raw materials.

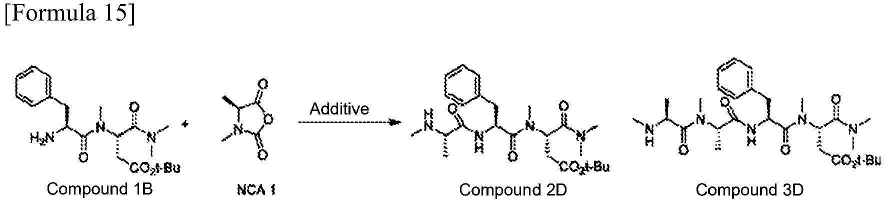

### Run 1

To a reaction vessel, tert-butyl (S)-3-((S)-2-amino-N-methyl-3-phenylpropanamide)-4-(dimethylamino)-4-oxobutanoate (51.7 mg), MeTHF (0.5 mL), and (S)-3,4-dimethyloxazolidine-2,5-dione (19.4 mg) were sequentially added and allowed to react at room temperature for 3 hours. The resulting reaction mixture was analyzed by HPLC analysis condition method 1.

### Run 2

To a reaction vessel, tert-butyl (S)-3-((S)-2-amino-N-methyl-3-phenylpropanamide)-4-(dimethylamino)-4-oxobutanoate (50.9 mg), MeTHF (0.5 mL), La(OTf)₃ (26.8 mg), and (S)-3,4-dimethyloxazolidine-2,5-dione (19.4 mg) were sequentially added and allowed to react at room temperature for 32 hours. The resulting reaction mixture was analyzed by HPLC analysis condition method 1.

For the results of Runs 1 to 2, Table 5 shows the peak area ratios (%) of compound 1B (raw material), compound 2D (target), and compound 3D (impurity) in each reaction mixture.

HPLC analysis data for each compound are shown below.
Compound 2D: retention time 2.7 min, LCMS m/z 378 [M+H]⁺
Compound 3D: retention time 3.7 min, LCMS m/z 463 [M+H]⁺

**[Table 5]**

| Run | Additive (equivalent) | Compound 1B | Compound 2D | Compound 3D |
|---|---|---|---|---|
| 1 | None | 0.16 | 82.81 | 17.03 |
| 2 | La(OTf)₃ (0.33 eq.) | 0.19 | 97.88 | 1.93 |

As is clear from Table 5, a large amount of over-elongation product (compound 3D), which is an impurity, was generated when no additive was added (Run 1). In contrast, when the Lewis acid was added, the generation of the over-elongation product (compound 3D), which is an impurity, was reduced, and compound 2D (target) was selectively obtained (Run 2).

### Example 5:

### Synthesis of tert-butyl (S)-4-(dimethylamino)-3-((S)-N-methyl-2-((S)-2-(methylamino)-3-phenylpropanamide)-3-phenylpropanamide)-4-oxobutanoate (compound 2E)

The reaction conditions for the synthesis of tert-butyl (S)-4-(dimethylamino)-3-((S)-N-methyl-2-((S)-2-(methylamino)-3-phenylpropanamide)-3-phenylpropanamide)-4-oxobutanoate (compound 2E) were studied using tert-butyl (S)-3-((S)-2-amino-N-methyl-3-phenylpropanamide)-4-(dimethylamino)-4-oxobutanoate (compound 1B) and (S)-4-benzyl-3-methyloxazolidine-2,5-dione (NCA2) as raw materials.

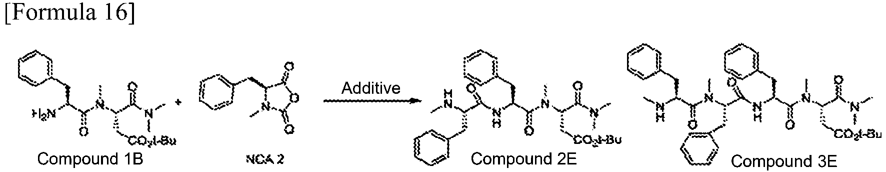

### Run 1

To a reaction vessel, tert-butyl (S)-3-((S)-2-amino-N-methyl-3-phenylpropanamide)-4-(dimethylamino)-4-oxobutanoate (51.4 mg), MeTHF (0.5 mL), and (S)-4-benzyl-3-methyloxazolidine-2,5-dione (30.4 mg) were sequentially added and allowed to react at room temperature for 22 hours. The resulting reaction mixture was analyzed by HPLC analysis condition method 1.

### Run 2

To a reaction vessel, tert-butyl (S)-3-((S)-2-amino-N-methyl-3-phenylpropanamide)-4-(dimethylamino)-4-oxobutanoate (51.3 mg), MeTHF (0.5 mL), CaI₂ (22.4 mg), and (S)-4-benzyl-3-methyloxazolidine-2,5-dione (30.3 mg) were sequentially added and allowed to react at room temperature for 22 hours. The resulting reaction mixture was analyzed by HPLC analysis condition method 1.

For the results of Runs 1 to 2, Table 6 shows the peak area ratios (%) of compound 1B (raw material), compound 2E (target), and compound 3E (impurity) in each reaction mixture.

HPLC analysis data for each compound are shown below.
Compound 2E: retention time 2.7 min, LCMS m/z 539 [M+H]⁺
Compound 3E: retention time 3.7 min, LCMS m/z 700 [M+H]⁺

**[Table 6]**

| Run | Additive (equivalent) | Compound 1B | Compound 2E | Compound 3E |
|---|---|---|---|---|
| 1 | None | 0.12 | 97.65 | 2.23 |
| 2 | CaI₂ (0.5 eq.) | 0.75 | 98.70 | 0.55 |

As is clear from Table 6, a large amount of over-elongation product (compound 3E), which is an impurity, was generated when no additive was added (Run 1). In contrast, when the Lewis acid was added, the generation of the over-elongation product (compound 3E), which is an impurity, was reduced, and compound 2E (target) was selectively obtained (Run 2).

### Example 6:

### Synthesis of benzyl ((S)-2-(methylamino)butanoyl)-L-isoleucinate (compound 2F)

The reaction conditions for the synthesis of benzyl ((S)-2-(methylamino)butanoyl)-L-isoleucinate (compound 2F) were studied using benzyl L-isoleucinate (compound 1C) and (S)-4-ethyl-3-methyloxazolidine-2,5-dione (NCA4) as raw materials.

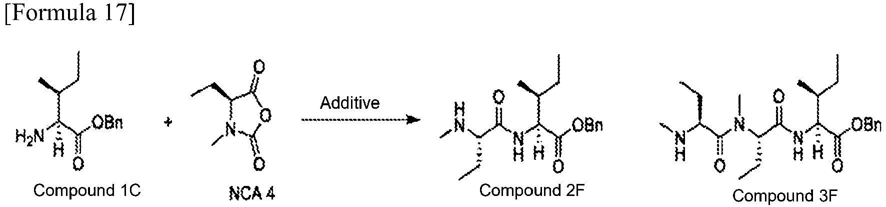

### Run 1

To a reaction vessel, benzyl L-isoleucinate (51.2 mg), MeTHF (0.5 mL), and (S)-4-ethyl-3-methyloxazolidine-2,5-dione (36.0 mg) were sequentially added and allowed to react at room temperature for 3 hours. The resulting reaction mixture was analyzed by HPLC analysis condition method 1.

### Run 2

To a reaction vessel, benzyl L-isoleucinate (50.2 mg), MeTHF (0.5 mL), CaI₂ (35.4 mg) and (S)-4-ethyl-3-methyloxazolidine-2,5-dione (36.1 mg) were sequentially added and allowed to react at room temperature for 22 hours. The resulting reaction mixture was analyzed by HPLC analysis condition method 1.

For the results of Runs 1 to 2, Table 7 shows the peak area ratios (%) of compound 1C (raw material), compound 2F (target), and compound 3F (impurity) in each reaction mixture.

HPLC analysis data for each compound are shown below.
Compound 2F: retention time 2.9 min, LCMS m/z 321 [M+H]⁺
Compound 3F: retention time 3.2 min, LCMS m/z 420 [M+H]⁺

**[Table 7]**

| Run | Additive (equivalent) | Compound 1C | Compound 2F | Compound 3F |
|---|---|---|---|---|
| 1 | None | 1.80 | 93.98 | 4.23 |
| 2 | CaI₂ (0.5 eq.) | 1.77 | 98.00 | 0.23 |

As is clear from Table 7, a large amount of over-elongation product (compound 3F), which is an impurity, was generated when no additive was added (Run 1). In contrast, when the Lewis acid was added, the generation of the over-elongation product (compound 3F), which is an impurity, was reduced, and compound 2F (target) was selectively obtained (Run 2).

### Example 7:

### Synthesis of tert-butyl L-alanyl-L-phenylalaninate (compound 2G)

The reaction conditions for the synthesis of tert-butyl L-alanyl-L-phenylalaninate (compound 2G) were studied using tert-butyl L-phenylalaninate (compound 1A) and (S)-4-methyloxazolidine-2,5-dione (NCA5) as raw materials.

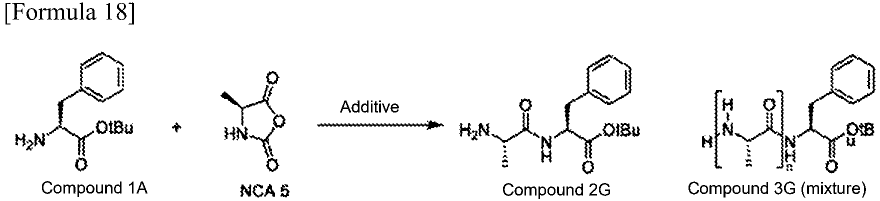

In the formula, n is 2 or more and 4 or less.

### Run 1

To a reaction vessel, tert-butyl L-phenylalaninate (51.1 mg), MeTHF (0.5 mL), and (S)-4-methyloxazolidine-2,5-dione (27.8 mg) were sequentially added and allowed to react at room temperature for 1 hour. The resulting reaction mixture was analyzed by HPLC analysis condition method 1.

### Run 2

To a reaction vessel, tert-butyl L-phenylalaninate (51.3 mg), MeTHF (0.5 mL), La(OTf)₃ (86.6 mg) and (S)-4-methyloxazolidine-2,5-dione (40.2 mg) were sequentially added and allowed to react at room temperature for 7 hours. The resulting reaction mixture was analyzed by HPLC analysis condition method 1.

For the results of Runs 1 to 2, Table 8 shows the peak area ratios (%) of compound 1A (raw material), compound 2G (target), and compound 3G (impurity) in each reaction mixture.

HPLC analysis data for each compound are shown below.
Compound 2G: retention time 2.55 min, LCMS m/z 293 [M+H]⁺
Compound 3G: retention time 3.60 min, LCMS m/z 364 [M+H]⁺ (n=2), 435 [M+H]⁺ (n=3), 506 [M+H]⁺ (n=4)

**[Table 8]**

| Run | Additive (equivalent) | Compound 1A | Compound 2G | Compound 3G |
|---|---|---|---|---|
| 1 | None | 70.16 | 15.50 | 14.34 |
| 2 | La(OTf)₃ (0.66 eq.) | 23.59 | 66.68 | 9.02 |

As is clear from Table 8, a large amount of over-elongation product (compound 3G), which is an impurity, was generated when no additive was added (Run 1). In contrast, when the Lewis acid was added, the generation of the over-elongation product (compound 3G), which is an impurity, was reduced, and compound 2G (target) was selectively obtained (Run 2).

### Example 8:

### Synthesis of tert-butyl L-valyl-L-phenylalaninate (compound 2H)

The reaction conditions for the synthesis of tert-butyl L-valyl-L-phenylalaninate (compound 2H) were studied using tert-butyl L-phenylalaninate (compound 1A) and (S)-4-isopropyloxazolidine-2,5-dione (NCA6) as raw materials.

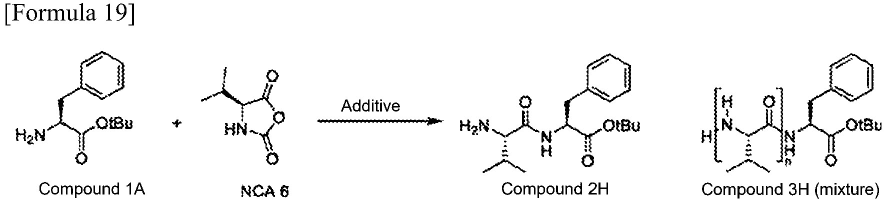

In the formula, n is 2 or more and 4 or less.

### Run 1

To a reaction vessel, tert-butyl L-phenylalaninate (50.8 mg), MeTHF (0.5 mL), and (S)-4-isopropyloxazolidine-2,5-dione (34.8 mg) were sequentially added and allowed to react at room temperature for 1 hour. The resulting reaction mixture was analyzed by HPLC analysis condition method 1.

### Run 2

To a reaction vessel, tert-butyl L-phenylalaninate (50.4 mg), MeTHF (0.5 mL), CaI₂ (34.2 mg) and (S)-4-isopropyloxazolidine-2,5-dione (34.8 mg) were sequentially added and allowed to react at room temperature for 23 hours. The resulting reaction mixture was analyzed by HPLC analysis condition method 1.

For the results of Runs 1 to 2, Table 9 shows the peak area ratios (%) of compound 1A (raw material), compound 2H (target), and compound 3H (impurity) in each reaction mixture.

HPLC analysis data for each compound are shown below.
Compound 2H: retention time 2.8 min, LCMS m/z 321 [M+H]⁺
Compound 3H: retention time 3.0 min, LCMS m/z 420 [M+H]⁺(n=2), retention time 3.2 min, LCMS m/z 519 [M+H]⁺ (n=3), retention time 3.3 min, LCMS m/z 618 [M+H]⁺ (n=4)

**[Table 9]**

| Run | Additive (equivalent) | Compound 1A | Compound 2H | Compound 3H |
|---|---|---|---|---|
| 1 | None | 27.84 | 47.88 | 24.28 |
| 2 | CaI₂ (0.5 eq.) | 14.52 | 74.19 | 6.53 |

As is clear from Table 9, a large amount of over-elongation product (compound 3H), which is an impurity, was generated when no additive was added (Run 1). In contrast, when the Lewis acid was added, the generation of the over-elongation product (compound 3H), which is an impurity, was reduced, and compound 2H (target) was selectively obtained (Run 2).

### Example 9:

### Synthesis of tert-butyl L-tyrosyl-L-phenylalaninate (compound 2I)

The reaction conditions for the synthesis of tert-butyl L-tyrosyl-L-phenylalaninate (compound 2I) were studied using tert-butyl L-phenylalaninate (compound 1A) and (S)-4-hydroxybenzyloxazolidine-2,5-dione (NCA7) as raw materials.

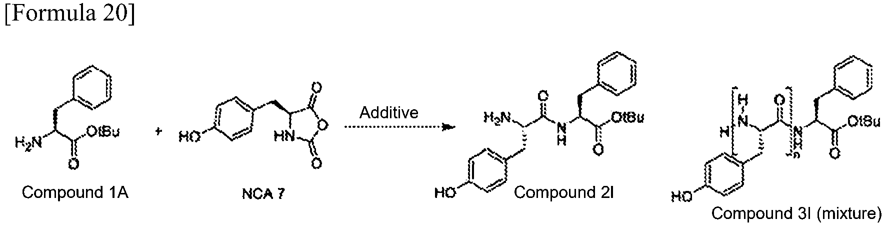

In the formula, n is 2 or more and 4 or less.

### Run 1

To a reaction vessel, tert-butyl L-phenylalaninate (50.8 mg), MeTHF (0.5 mL), and (S)-4-hydroxybenzyloxazolidine-2,5-dione (49.8 mg) were sequentially added and allowed to react at room temperature for 1 hour. The resulting reaction mixture was analyzed by HPLC analysis condition method 1.

### Run 2

To a reaction vessel, tert-butyl L-phenylalaninate (50.4 mg), MeTHF (0.5 mL), La(OTf)₃ (44.4 mg) and (S)-4-hydroxybenzyloxazolidine-2,5-dione (50.2 mg) were sequentially added and allowed to react at room temperature for 23 hours. The resulting reaction mixture was analyzed by HPLC analysis condition method 1.

For the results of Runs 1 to 2, Table 10 shows the peak area ratios (%) of compound 1A (raw material), compound 2I (target), and compound 3I (impurity) in each reaction mixture.

HPLC analysis data for each compound are shown below.
Compound 2I: retention time 2.8 min, LCMS m/z 385 [M+H]⁺
Compound 3I: retention time 3.0 min, LCMS m/z 548 [M+H]⁺ (n=2), retention time 3.1 min, LCMS m/z 711 [M+H]⁺ (n=3), retention time 3.2 min, LCMS m/z 874 [M+H]⁺ (n=4)

**[Table 10]**

| Run | Additive (equivalent) | Compound 1A | Compound 21 | Compound 31 |
|---|---|---|---|---|
| 1 | None | 15.13 | 49.65 | 35.23 |
| 2 | La(OTf)₃ (0.33 eq.) | 10.51 | 71.22 | 17.31 |

As is clear from Table 10, a large amount of over-elongation product (compound 3I), which is an impurity, was generated when no additive was added (Run 1). In contrast, when the Lewis acid was added, the generation of the over-elongation product (compound 3I), which is an impurity, was reduced, and compound 2I (target) was selectively obtained (Run 2).

### Example 10:

### Synthesis of tert-butyl N-((S)-2-((S)-N-ethyl-1-(methyl-L-alanyl)azetidine-2-carboxamide)-3-(p-tolyl)propanoyl)-N-methylglycinate (compound 2J)

N-(tert-Butoxycarbonyl)-N-methyl-L-alanine was reacted with thionyl chloride to afford (S)-3,4-dimethyloxazolidine-2,5-dione (NCA1). This was reacted with tert-butyl N-((S)-N-ethylazetidine-2-carboxamide)-3-(p-tolyl)propanoyl)-N-methylglycinate (compound 1D) without purification to synthesize tert-butyl N-((S)-2-((S)-N-ethyl-1-(methyl-L-alanyl)azetidine-2-carboxamide)-3-(p-tolyl)propanoyl)-N-methylglycinate (compound 2J). The reaction conditions for these reactions were studied.

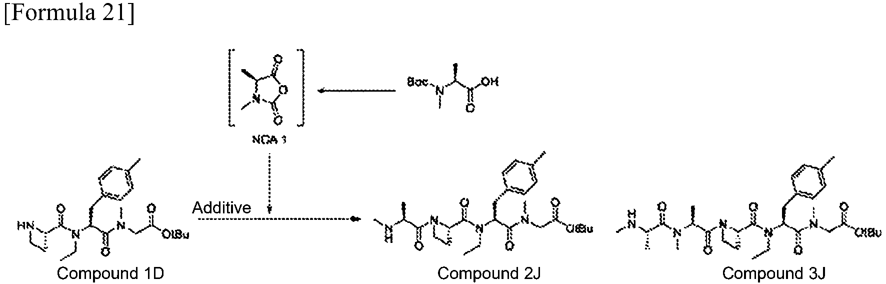

### Run 1

To a reaction vessel, N-(tert-butoxycarbonyl)-N-methyl-L-alanine (60.1 mg), THF (0.83 mL), and thionyl chloride (26 µL) were sequentially added and allowed to react at 40°C for 2 hours. The resulting reaction mixture was analyzed by HPLC analysis condition method 1 to confirm that (S)-3,4-dimethyloxazolidine-2,5-dione (NCA1) was produced. The reaction mixture was then concentrated to dryness.

To another reaction vessel, a solution of tert-butyl N-((S)-N-ethylazetidin-2-carboxamide)-3-(p-tolyl)propanoyl)-N-methylglycinate in MeTHF (218.4 mg, concentration: 46.7 w/w%) and MeTHF (1.1 mL) were added, and the previously prepared NCA1 was added, and the mixture was allowed to react at room temperature for 3 hours. The resulting reaction mixture was analyzed by HPLC analysis condition method 2.

### Run 2

To a reaction vessel, N-(tert-butoxycarbonyl)-N-methyl-L-alanine (60.6 mg), THF (0.83 mL), and thionyl chloride (26 µL) were sequentially added and allowed to react at 40°C for 2.5 hours. The resulting reaction mixture was analyzed by HPLC analysis condition method 1 to confirm that (S)-3,4-dimethyloxazolidine-2,5-dione (NCA1) was produced. The reaction mixture was then concentrated to dryness.

To another reaction vessel, a solution of tert-butyl N-((S)-N-ethylazetidin-2-carboxamide)-3-(p-tolyl)propanoyl)-N-methylglycinate in MeTHF (215.1 mg, concentration: 46.7 w/w%), MeTHF (1.1 mL), and La(OTf)₃ (58.2 mg) were added, and the previously prepared NCA1 was sequentially added, and the mixture was allowed to react at room temperature for 18 hours. The resulting reaction mixture was analyzed by HPLC analysis condition method 2.

### Run 3 (scale-up, post-processing method)

To a reaction vessel, N-(tert-butoxycarbonyl)-N-methyl-L-alanine (1.25 g), THF (16.5 mL), and thionyl chloride (0.52 mL) were sequentially added and allowed to react at 40°C for 2 hours. The resulting reaction mixture was analyzed by HPLC analysis condition method 1 to confirm that (S)-3,4-dimethyloxazolidine-2,5-dione (NCA1) was produced. The reaction mixture was then concentrated, MeTHF (6 mL) was added to the residue, and then the mixture was further concentrated. This procedure was repeated one more time. To the resulting residue, MeTHF (5 mL) was added to dissolve.

To another reaction vessel, a solution of tert-butyl N-((S)-N-ethylazetidin-2-carboxamide)-3-(p-tolyl)propanoyl)-N-methylglycinate in MeTHF (4.29 g, concentration: 46.7 w/w%), MeTHF (15 mL), and La(OTf)₃ (1.15 g) were added, and the previously prepared solution of NCA1 in MeTHF was sequentially added, and the mixture was stirred at 40°C for 5 hours, and then at room temperature for 16 hours. The resulting reaction mixture was analyzed by HPLC analysis condition method 2. The resulting reaction mixture was washed twice with 5% aqueous disodium ethylenediaminetetraacetate solution (24 mL) and further with 5% aqueous potassium carbonate solution (24 mL). The resulting organic layer was concentrated to afford compound 2J (1.80 g, yellow oil).

For the results of Runs 1 to 3, Table 11 shows the peak area ratios (%) of compound 1D (raw material), compound 2J (target), and compound 3J (impurity) in each reaction mixture.

HPLC analysis data for each compound are shown below.
Compound 2J: retention time 7.6 min, LCMS m/z 503 [M+H]⁺
Compound 3J: retention time 8.0 min, LCMS m/z 588 [M+H]⁺

**[Table 11]**

| Run | Additive (equivalent) | Compound 1D | Compound 2J | Compound 3J |
|---|---|---|---|---|
| 1 | None | 6.20 | 85.92 | 7.88 |
| 2 | La(OTf)₃ (0.4 eq.) | 0.18 | 99.34 | 0.18 |
| 3 | La(OTf)₃ (0.4 eq.) | 0.17 | 99.14 | 0.69 |

As is clear from Table 11, it was found that excellent selectivity was obtained not only when purified NCA was used, but also when a high-purity NCA compound was prepared by the above reaction conditions and used without purification in the reaction.

### Example 11:

### Synthesis of tert-butyl methyl-L-phenylalanyl-L-phenylalaninate (compound 2B)

N-(tert-Butoxycarbonyl)-N-methyl-L-phenylalanine was reacted with thionyl chloride to afford (S)-4-benzyl-3-methyloxazolidine-2,5-dione (NCA2). This was reacted with tert-butyl L-phenylalaninate (compound 1A) without purification to synthesize tert-butyl methyl-L-phenylalanyl-L-phenylalaninate (compound 2B). The reaction conditions for these reactions were studied.

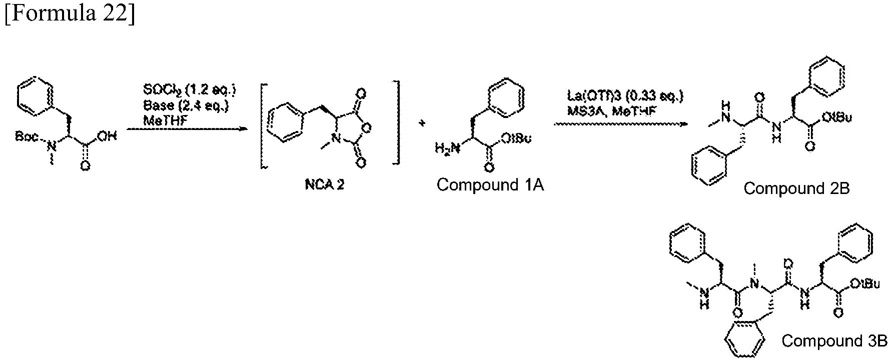

### Runs 1 to 5

To a reaction vessel, N-(tert-butoxycarbonyl)-N-methyl-L-phenylalanine (60 mg), MeTHF (0.48 mL), a base (2.4 eq.), and thionyl chloride (16 µL) were sequentially added and allowed to react at room temperature to 40°C for 2 to 3 hours. The resulting reaction mixture was analyzed by HPLC analysis condition method 1 to confirm that NCA2 was produced. To another reaction vessel, tert-butyl L-phenylalaninate (40 mg), La(OTf)₃ (0.33 eq.), molecular sieve 3A (1 to 2 w/w%) and the resulting reaction solution of (S)-4-benzyl-3-methyloxazolidine-2,5-dione were sequentially added and allowed to react at room temperature for 22 hours. The resulting reaction mixture was analyzed by HPLC analysis condition method 1.

For the results of Runs 1 to 5, Table 12 shows the reaction conversion and selectivity calculated from the peak areas of compound 1A (raw material), compound 2B (target), and compound 3B (impurity) in each reaction mixture. The reaction conversion is calculated by the calculation formula "(compound 2B + compound 3B)/(compound 1A + compound 2B + compound 3B) × 100%". The selectivity (X/Y) was calculated by the calculation formulas "X = compound 2B/(compound 2B + compound 3B) × 100%, Y = compound 3B/(compound 2B + compound 3B) × 100%".

HPLC analysis data for each compound are shown below.
Compound 2B: retention time 3.2 min, LCMS m/z 383 [M+H]⁺
Compound 3B: retention time 3.7 min, LCMS m/z 544 [M+H]⁺

**[Table 12]**

| Run | Base | Reaction conversion | Selectivity |
|---|---|---|---|
| Example 2 Run 2 | (using isolated and purified NCA) | 99.7 | 99.7/0.3 |
| 1 | DIPEA | 84.9 | 99.6/0.4 |
| 2 | 1-methylpiperidine | 56.2 | >99.1/0.1 |
| 3 | 4-ethylmorpholine | 52.5 | >99.1/0.1 |
| 4 | NMM | 53.4 | >99.1/0.1 |
| 5 | 2,4,6-collidine | 26.0 | >99.1/0.1 |

### Example 12:

### Synthesis of tert-butyl methyl-L-phenylalanyl-L-phenylalaninate (compound 2B)

N-(tert-Butoxycarbonyl)-N-methyl-L-phenylalanine was reacted with thionyl chloride to afford (S)-4-benzyl-3-methyloxazolidine-2,5-dione (NCA2). This was reacted with tert-butyl L-phenylalaninate (compound 1A) without purification to synthesize tert-butyl methyl-L-phenylalanyl-L-phenylalaninate (compound 2B). The reaction conditions for these reactions were studied.

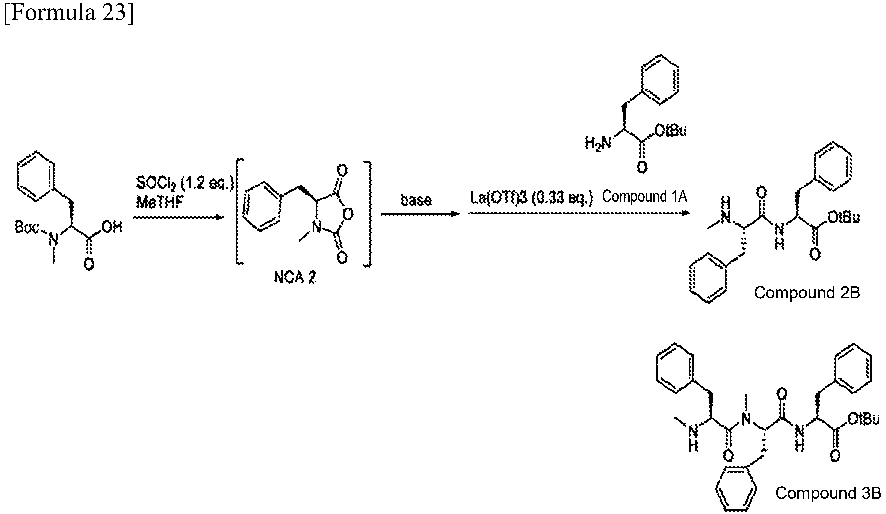

### Runs 1 to 9

To a reaction vessel, N-(tert-butoxycarbonyl)-N-methyl-L-phenylalanine (60 mg), THF or MeTHF (0.48 mL), and thionyl chloride (16 µL) were sequentially added and allowed to react at 40°C for 2.5 hours. The resulting reaction mixture was analyzed by HPLC analysis condition method 1 to confirm that NCA2 was produced, and then a base (1.0 or 2.0 eq.) was added. Subsequently, La(OTf)₃ (0.33 eq.) and tert-butyl L-phenylalaninate (40 mg) were sequentially added and the mixture was allowed to react at room temperature for 20 hours. The resulting reaction mixture was analyzed by HPLC analysis condition method 1.

For the results of Runs 1 to 9, Table 13 shows the reaction conversion and selectivity calculated from the peak areas of compound 1A (raw material), compound 2B (target), and compound 3B (impurity) in each reaction mixture. The reaction conversion is calculated by the calculation formula "(compound 2B + compound 3B)/(compound 1A + compound 2B + compound 3B) × 100%". The selectivity (X/Y) is calculated by the calculation formulas "X = compound 2B/(compound 2B + compound 3B) × 100%, Y = compound 3B/(compound 2B + compound 3B) × 100%".

HPLC analysis data for each compound are shown below.
Compound 2B: retention time 3.2 min, LCMS m/z 383 [M+H]⁺
Compound 3B: retention time 3.7 min, LCMS m/z 544 [M+H]⁺

**[Table 13]**

| Run | Base (equivalent) | Solvent | Reaction conve rsion | Selectivity |
|---|---|---|---|---|
| Example 2 Run 2 | (using isolated and purified NCA) | MeTHF | 99.7 | 99.7/0.3 |
| 1 | K₂CO₃ (2 eq.) | MeTHF | 97.3 | 99.7/0.3 |
| 2 | KHCO₃ (2 eq.) | MeTHF | 4.0 | >99.1/0.1 |
| 3 | CaO (2 eq.) | MeTHF | 97.2 | >99.1/0.1 |
| 4 | MgO (1 eq.) | MeTHF | <1 | >99.1/0.1 |
| 5 | Cs₂CO₃ (2 eq.) | MeTHF | 96.3 | 99.2/0.8 |
| 6 | CsHCO₃ (2 eq.) | MeTHF | 93.1 | 99.8/0.2 |
| 7 | K₃PO₄ (1 eq.) | THF | <1 | >99.1/0.1 |
| 8 | K₂HPO₄ (2 eq.) | THF | 63.1 | >99.1/0.1 |
| 9 | NaHCO₃ (2 eq.) | THF | 81.6 | >99.1/0.1 |

### Synthetic Example 1:

### Synthesis of (S)-3,4-dimethyloxazolidine-2,5-dione (NCA1)

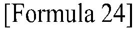

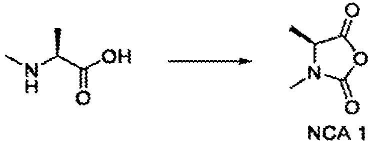

To a reaction vessel, methyl-L-alanine (4.00 g), triphosgene (4.61 g), and tetrahydrofuran (80 mL) were added at room temperature. The outside temperature was raised to 50°C and the mixture was reacted for 4 hours. The reaction solvent was concentrated under reduced pressure and the concentrate was purified by column (n-heptane/ethyl acetate) and concentrated under reduced pressure to afford NCA1 (4.96 g).
Yield: 99%, HPLC purity: 100%

HPLC analysis data and NMR data for the compound are shown below.
NCA 1: retention time 1.1 min, LCMS m/z 130 [M+H]⁺
¹H-NMR (DMSO-D₆) δ: 4.40(1H, q, J = 7.1 Hz), 2.84 (3H, s), 1.39 (3H, d, J = 6.9 Hz).

### Synthetic Example 2:

### Synthesis of (S)-4-benzyl-3-methyloxazolidine-2,5-dione (NCA2)

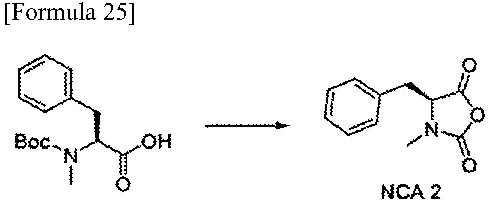

To a reaction vessel, N-(tert-butoxycarbonyl)-L-phenylalanine (4.54 g) and dichloromethane (8 mL) were added at room temperature. After the outside temperature was cooled to 0°C, triethylamine (2.3 mL) and thionyl chloride (1.3 mL) were sequentially added, then the temperature was raised to room temperature, and the mixture was allowed to react for an additional 0.5 hours. The reaction solvent was concentrated under reduced pressure and the concentrate was purified by column (n-heptane/ethyl acetate) and concentrated under reduced pressure to afford NCA2 (3.09 g).
Yield: 93%, HPLC purity: 100%

HPLC analysis data and NMR data for the compound are shown below.
NCA 2: retention time 3.2 min
¹H-NMR (DMSO-D₆) δ:7.32-7.27 (3H, m), 7.17-7.16 (2H, m), 4.70 (1H, t, J = 4.6 Hz), 3.24-3.21 (1H,m), 3.12-3.09 (1H, m), 2.90 (3H, s).

### Synthetic Example 3:

### Synthesis of 3-butyloxazolidine-2,5-dione (NCA3)

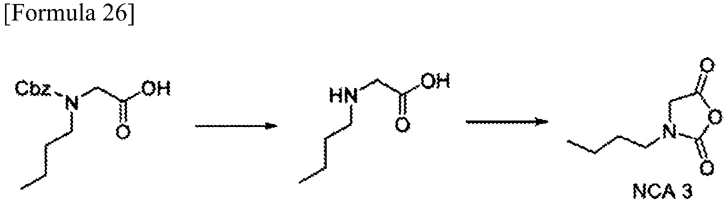

To a reaction vessel, N-(benzyloxycarbonyl)-N-butylglycine (5.41 g) and tetrahydrofuran (80 mL) were added at room temperature, then the vesselwas purged with nitrogen, and 5% Pd/C (0.55 g) was added. Subsequently, the atmosphere was replaced with hydrogen, and the mixture was reacted for 4 hours under the hydrogen atmosphere of 1 atm. To the reaction mixture, 1N aqueous sodium hydroxide solution (20 mL) was added, and Pd/C was filtered. To the filtrate, 2N hydrochloric acid (10 mL) was added, and the mixture was concentrated under reduced pressure to dryness to obtain the crude product of butylglycine. To the crude product of the resulting butylglycine, triphosgene (3.04 g) and tetrahydrofuran (53 mL) were added. The outside temperature was raised to 50°C and the mixture was reacted for 1 hour. The reaction solvent was concentrated under reduced pressure and the concentrate was purified by column (n-heptane/ethyl acetate) and concentrated under reduced pressure to afford NCA3 (1.77 g).
Yield: 55% (2 steps), HPLC purity: 89.8%

HPLC analysis data and NMR data for the compound are shown below.
NCA 3: retention time 2.4 min, LCMS m/z 132 [M-CO₂+H₂O+H]⁺
¹H-NMR (DMSO-D₆) δ: 4.25(2H, s), 3.27 (2H, t, J = 7.2 Hz), 1.52-1.47 (2H, m), 1.34-1.27 (2H, m), 0.89(3H, t, J = 7.4 Hz).

### Synthetic Example 4:

### Synthesis of (S)-4-ethyl-3-methyloxazolidine-2,5-dione (NCA4)

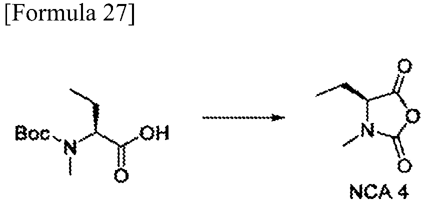

To a reaction vessel, (S)-2-((tert-butoxycarbonyl)methyl)amino)butyric acid (3.00 g) and dichloromethane (12 mL) were added at room temperature. After the outside temperature was cooled to 0°C, triethylamine (1.93 mL) and thionyl chloride (1.1 mL) were sequentially added, and the mixture was allowed to react for 3 hours. The reaction solvent was concentrated under reduced pressure and the concentrate was purified by column (n-heptane/ethyl acetate) and concentrated under reduced pressure to afford NCA4 (1.16 g).
Yield: 59%, HPLC purity: 100%

HPLC analysis data and NMR data for the compound are shown below.
NCA 4: retention time 1.6 min
¹H-NMR (DMSO-D₆) δ:4.43-4.41 (1H, m), 2.85 (3H, s), 1.92-1.79 (2H, m), 0.82 (3H, t, J = 7.4 Hz).

### Synthetic Example 5:

### tert-Butyl L-phenylalaninate (compound 1A)

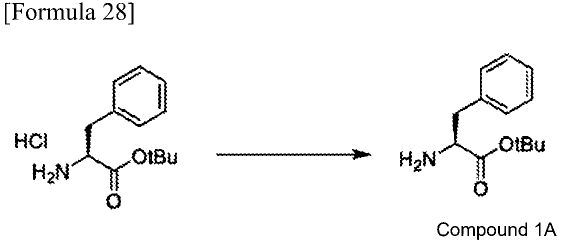

To a reaction vessel, L-phenylalanine tert-butyl hydrochloride (5.02 g), ethyl acetate (50 mL), and 5% aqueous sodium hydrogen carbonate solution (39 mL) were added at room temperature, and the mixture was stirred for 0.5 hours. The aqueous layer was discarded and the organic layer was washed twice with 1% saline (50 mL). The resulting organic layer was concentrated under reduced pressure to obtain compound 1A (3.76 g).
Yield: 88%, HPLC purity: 100%

HPLC analysis data for the compound are shown below.
Compound 1A: retention time 2.2 min, LCMS m/z 166 [M-tBu+H]⁺

### Synthetic Example 6:

### tert-butyl (S)-3-((S)-2-amino-N-methyl-3-phenylpropanamide)-4-(dimethylamino)-4-oxobutanoate (compound 1B)

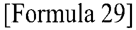

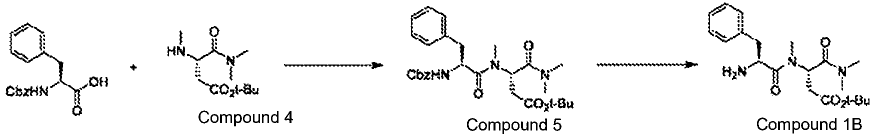

To a reaction vessel, a solution of tert-butyl (S)-4-(dimethylamino)-3-(methylamino)-4-oxobutanoate (compound 4) in MeTHF (8.44 g, 30.2 w/w%), N-benzyloxycarbonyl-L-phenylalanine (3.33 g), MeTHF (20 mL), acetonitrile (5 mL), N,N-diisopropylethylamine (5 mL), and HATU (5.48 g) were added at room temperature, and the mixture was allowed to react for 1 hour. Subsequently, 5% aqueous potassium carbonate solution (25 mL) and N-methylimidazole (0.44 mL) were added to the reaction mixture, and the resulting mixture was stirred for 0.5 hours. The organic layer was washed twice with 5% aqueous sodium hydrogen sulfate solution (25 mL) and then twice with 5% aqueous potassium carbonate solution (25 mL). The organic layer was concentrated under reduced pressure to afford a crude product of compound 5. To a resulting compound 5, MeTHF (25 mL) was added, then the vessel was purged with nitrogen, and 5% Pd/C (0.60 g) was added. The atmosphere was replaced with hydrogen, and the mixture was reacted for 3 hours under the hydrogen atmosphere of 1 atm. Subsequently, Pd/C was filtered, and the resulting filtrate was concentrated under reduced pressure to obtain compound 1B (4.17 g).
Yield: 100% (2 steps), HPLC purity: 95.7%

HPLC analysis data for the compound are shown below.
Compound 1B: retention time 2.2 min, LCMS m/z 378 [M+H]⁺

tert-Butyl N-((S)-N-ethylazetidine-2-carboxamide)-3-(p-tolyl)propanoyl)-N-methylglycinate (compound 1D), and tert-butyl (S)-4-(dimethylamino)-3-(methylamino)-4-oxobutanoate (compound 4) were synthesized by the method described in International Publication No. WO 2022/234864.

### Example 13:

### Synthesis of tert-butyl methyl-L-alanyl-L-phenylalaninate (compound 2A)

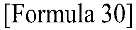

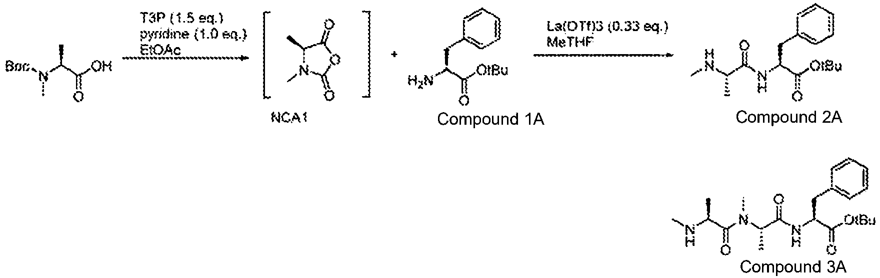

To a reaction vessel, N-(tert-butoxycarbonyl)-N-methyl-L-alanine (51.7 mg), ethyl acetate (1.5 mL), a solution of propylphosphonic anhydride in ethyl acetate (0.27 mL), and pyridine (20 µL) were sequentially added and allowed to react at 40°C for 4 hours. The resulting reaction mixture was analyzed by HPLC analysis condition method 1 to confirm that (S)-3,4-dimethyloxazolidine-2,5-dione (NCA1) was produced. Then, a 5% aqueous potassium carbonate solution (1 mL) was added to the reaction mixture, and the resulting mixture was stirred at room temperature for 5 minutes. The resulting organic layer was concentrated to dryness. To another reaction vessel, tert-butyl L-phenylalaninate (48.9 mg), MeTHF (0.49 mL), La(OTf)₃ (42.5 mg) and the previously prepared (S)-3,4-dimethyloxazolidine-2,5-dione (NCA1) were added and allowed to react at room temperature for 3 hours. The resulting reaction mixture was analyzed by HPLC analysis condition method 1.

**[Table 14]**

| Run | Additive (equivalent) | Reaction conversion (%) | Selectivity |
|---|---|---|---|
| Example 1 Run 1 | None (using isolated and purified NCA) | 95.6 | 94.1/5.9 |
| Example 13 | La(OTf)₃ (0.33 eq.) | 67.8 | 99.8/0.2 |

Table 14 shows the reaction conversion and selectivity calculated from the peak areas of compound 1A (raw material), compound 2A (target), and compound 3A (impurity) in each reaction mixture. The reaction conversion is calculated by the calculation formula "(compound 2A + compound 3A)/(compound 1A + compound 2A + compound 3A) × 100%". The selectivity (X/Y) is calculated by the calculation formulas "X = compound 2A/(compound 2A + compound 3A) × 100%, Y = compound 3A/(compound 2A + compound 3A) × 100%".

HPLC analysis data for each compound are shown below.
Compound 1A: retention time 2.4 min, LCMS m/z 222 [M+H]⁺
Compound 2A: retention time 2.6 min, LCMS m/z 307 [M+H]⁺
Compound 3A: retention time 2.8 min, LCMS m/z 392 [M+H]⁺

### Example 14:

### Synthesis of tert-butyl methyl-L-alanyl-L-phenylalaninate (Compound 2A)

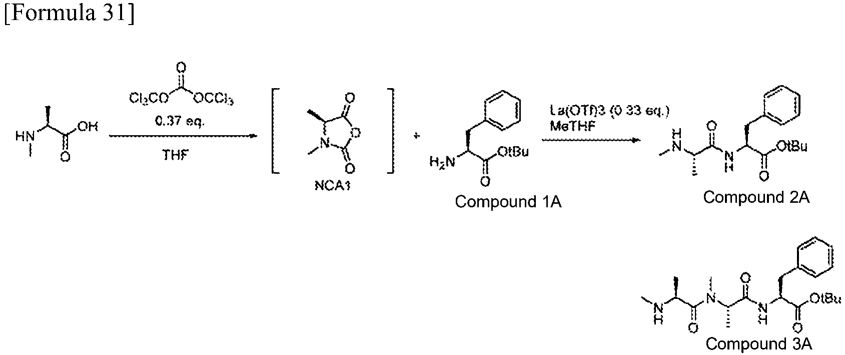

To a reaction vessel, N-methyl-L-alanine (26.2 mg), tetrahydrofuran (1 mL), and triphosgene (25.2 mg) were sequentially added and allowed to react at 40°C for 2 hours and then at 50°C for 2 hours. The resulting reaction mixture was analyzed by HPLC analysis condition method 1 to confirm that (S)-3,4-dimethyloxazolidine-2,5-dione (NCA1) was produced. The reaction mixture was then concentrated to dryness. To another reaction vessel, tert-butyl L-phenylalaninate (45.8 mg), MeTHF (0.45 mL), La(OTf)₃ (40.0 mg) and the previously prepared (S)-3,4-dimethyloxazolidine-2,5-dione (NCA1) were added and allowed to react at room temperature for 18 hours. The resulting reaction mixture was analyzed by HPLC analysis condition method 1.

**[Table 15]**

| Run | Additive (equivalent) | Reaction conversion (%) | Selectivity |
|---|---|---|---|
| Example 1 Run 1 | None (using isolated and purified NCA) | 95.6 | 94.1/5.9 |
| Example 14 | La(OTf)₃ (0.33 eq.) | 77.4 | 99.8/0.2 |

Table 15 shows the reaction conversion and selectivity calculated from the peak areas of compound 1A (raw material), compound 2A (target), and compound 3A (impurity) in each reaction mixture. The reaction conversion is calculated by the calculation formula "(compound 2A + compound 3A)/(compound 1A + compound 2A + compound 3A) × 100%". The selectivity (X/Y) is calculated by the calculation formulas "X = compound 2A/(compound 2A + compound 3A) × 100%, Y = compound 3A/(compound 2A + compound 3A) × 100%".

HPLC analysis data for each compound are shown below.
Compound 1A: retention time 2.4 min, LCMS m/z 222 [M+H]⁺
Compound 2A: retention time 2.6 min, LCMS m/z 307 [M+H]⁺
Compound 3A: retention time 2.8 min, LCMS m/z 392 [M+H]⁺

### Example 15:

### Synthesis of tert-butyl methyl-L-phenylalanyl-L-phenylalaninate (compound 2B)

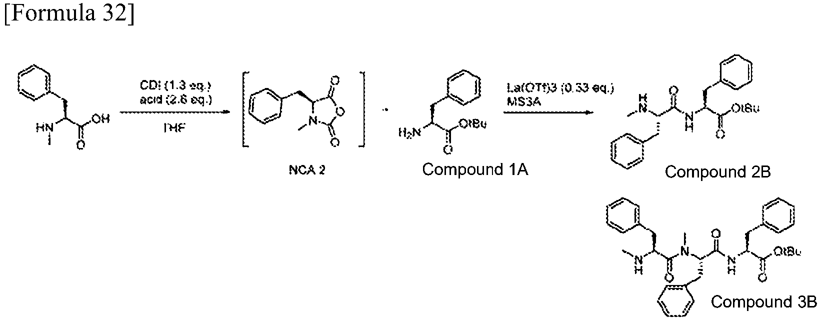

### Run 1

To a reaction vessel, N-methyl-L-phenylalanine (50.1 mg), carbonyl diimidazole (45.3 mg), tetrahydrofuran (0.5 mL), and trifluoromethanesulfonic acid (49 µL) were sequentially added and allowed to react at room temperature for 4 hours. The resulting reaction mixture was analyzed by HPLC analysis condition method 1 to confirm that (S)-4-benzyl-3-methyloxazolidine-2,5-dione (NCA2) was produced. To another reaction vessel, tert-butyl L-phenylalaninate (44.1 mg), La(OTf)₃ (41.7 mg), MS3A (51.2 mg), and the previously prepared (S)-4-benzyl-3-methyloxazolidine-2,5-dione (NCA2) were added and allowed to react at room temperature for 22 hours. The resulting reaction mixture was analyzed by HPLC analysis condition method 1.

### Run 2

To a reaction vessel, N-methyl-L-phenylalanine (50.6 mg), carbonyl diimidazole (46.3 mg), tetrahydrofuran (0.5 mL), and methanesulfonic acid (36 µL) were sequentially added and allowed to react at room temperature for 4 hours. The resulting reaction mixture was analyzed by HPLC analysis condition method 1 to confirm that (S)-4-benzyl-3-methyloxazolidine-2,5-dione (NCA2) was produced. To another reaction vessel, tert-butyl L-phenylalaninate (46.3 mg), La(OTf)₃ (41.2 mg), MS3A (60.9 mg), and the previously prepared (S)-4-benzyl-3-methyloxazolidine-2,5-dione (NCA2) were added and allowed to react at room temperature for 22 hours. The resulting reaction mixture was analyzed by HPLC analysis condition method 1.

**[Table 16]**

| Run | Acid (NCA preparation) | Additive (equivalent) | Reaction conversion (%) | Selectivity |
|---|---|---|---|---|
| Example 2 Run 1 | - | None (using isolated and purified NCA) | 99.9 | 98.2 /1.8 |
| Example 15 Run 1 | TfOH | La(OTf)₃ (0.33 eq.) | 92.6 | 99.8/0.2 |
| Example 15 Run 2 | MsOH | La(OTf)₃ (0.33 eq.) | 86.8 | 99.7/0.3 |

Table 16 shows the reaction conversion and selectivity calculated from the peak areas of compound 1A (raw material), compound 2B (target), and compound 3B (impurity) in each reaction mixture. The reaction conversion is calculated by the calculation formula "(compound 2B + compound 3B)/(compound 1A + compound 2B + compound 3B) × 100%". The selectivity (X/Y) is calculated by the calculation formulas "X = compound 2B/(compound 2B + compound 3B) × 100%, Y = compound 3B/(compound 2B + compound 3B) × 100%".

HPLC analysis data for each compound are shown below.
Compound 1A: retention time 2.4 min, LCMS m/z 222 [M+H]⁺
Compound 2B: retention time 2.6 min, LCMS m/z 383 [M+H]⁺
Compound 3B: retention time 2.8 min, LCMS m/z 544 [M+H]⁺

### Synthetic Example 7:

### Synthesis of 3-ethyl-4-(4-methylbenzyl)oxazolidine-2,5-dione (NCA8)

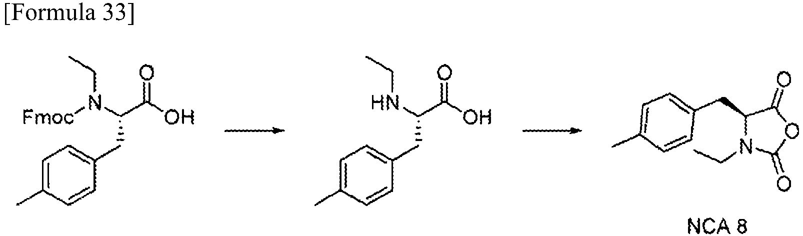

To a reaction vessel, (S)-2(((9H-fluoren-9-yl)ethoxy)carbonyl)(ethyl)amino)-3-(p-tolyl)propionic acid (5.02 g) and acetonitrile (25 mL) were added at room temperature followed by diazabicycloundecene (1.74 mL). The mixture was reacted for 2 hours, and then stored overnight. The precipitated solid was filtered and further washed with acetonitrile (5 mL) twice to afford a crude product of (S)-2-(ethylamino)-3-(p-tolyl)propionic acid. To the crude product of the resulting (S)-2-(ethylamino)-3-(p-tolyl)propionic acid, triphosgene (535 mg) and tetrahydrofuran (11 mL) were added. The outside temperature was raised to 50°C and the mixture was reacted for 4 hours. The reaction solvent was concentrated under reduced pressure and the concentrate was purified by column (n-heptane/ethyl acetate) and concentrated under reduced pressure to afford NCA8 (1.01 g).
Yield: 37% (2 steps), HPLC purity: 98.35%

HPLC analysis data and NMR data for the compound are shown below.
NCA 8: retention time 3.1 min
1H-NMR (CDCl₃) δ: 7.12 (2H, d, J = 8.0 Hz), 7.03 (2H, d, J = 8.0 Hz), 4.49 (1H, t, J = 4.9 Hz), 3.73 (1H, td, J = 14.5, 7.3 Hz), 3.16 (2H, d, J = 4.6 Hz), 3.13-3.09 (1H, m), 2.32 (3H, s), 1.18 (3H, t, J = 7.2 Hz).

### Example 16:

### Synthesis of tert-butyl ((S)-2-(ethylamino)-3-(p-tolyl)propanoyl-L-phenylalaninate (compound 2K)

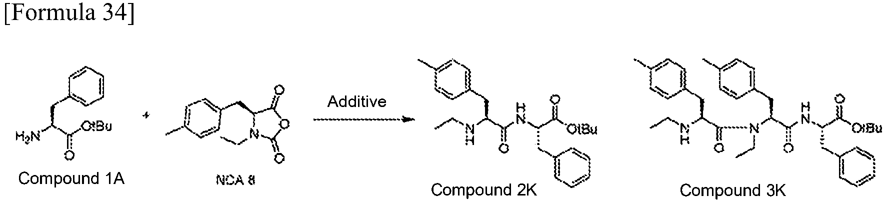

### Run 1

To a reaction vessel, tert-butyl L-phenylalaninate (50.1 mg), MS3A (51.4 mg), MeTHF (1 mL), and 3-ethyl-4-(4-methylbenzyl)oxazolidine-2,5-dione (68.1 mg) were sequentially added and allowed to react at 40°C for 10 hours and then at 50°C for 7 hours. The resulting reaction mixture was analyzed by HPLC analysis condition method 1.

### Run 2

To a reaction vessel, tert-butyl L-phenylalaninate (49.9 mg), MS3A (54.3 mg), MeTHF (1 mL), La(OTf)₃ (25.4 mg), and 3-ethyl-4-(4-methylbenzyl)oxazolidine-2,5-dione (69.2 mg) were sequentially added and allowed to react at 40°C for 10 hours and then at 50°C for 7 hours. The resulting reaction mixture was analyzed by HPLC analysis condition method 1.

### Run 3

To a reaction vessel, tert-butyl L-phenylalaninate (50.8 mg), MS3A (49.2 mg), MeTHF (1 mL), CaI₂ (19.6 mg), and 3-ethyl-4-(4-methylbenzyl)oxazolidine-2,5-dione (70.3 mg) were sequentially added and allowed to react at 40°C for 10 hours and then at 50°C for 7 hours. The resulting reaction mixture was analyzed by HPLC analysis condition method 1.

**[Table 17]**

| Run | Additive (equivalent) | Reaction conversion (%) | Selectivity |
|---|---|---|---|
| 1 | None | 99.1 | 99.1/0.9 |
| 2 | La(OTf)₃ (0.2 eq.) | 98.7 | >99.9/0.1 |
| 3 | CaI₂ (0.33 eq.) | 97.8 | 99.7/0.3 |

Table 17 shows the reaction conversion and selectivity calculated from the peak areas of compound 1A (raw material), compound 2K (target), and compound 3K (impurity) in each reaction mixture. The reaction conversion is calculated by the calculation formula "(compound 2K + compound 3K)/(compound 1A + compound 2K + compound 3K) × 100%". The selectivity (X/Y) is calculated by the calculation formulas "X = compound 2K/(compound 2K + compound 3K) × 100%, Y = compound 3K/(compound 2K + compound 3K) × 100%".

HPLC analysis data for each compound are shown below.
Compound 2K: retention time 2.9 min, LCMS m/z 411 [M+H]⁺
Compound 3K: retention time 3.7 min, LCMS m/z 600 [M+H]⁺

## Claims

1. A method for producing an amide group-containing compound, comprising step A of reacting an N-carboxyamino acid anhydride (NCA) with an amino group of an amino group-containing compound in a first solvent in the presence of a Lewis acid.

2. The method according to claim 1, wherein the Lewis acid is a metal salt.

3. The method according to claim 2, wherein the metal salt is selected from the group consisting of calcium trifluoromethanesulfonate, calcium iodide, zinc trifluoromethanesulfonate, lanthanum(III) trifluoromethanesulfonate, terbium(III) trifluoromethanesulfonate, holmium(III) trifluoromethanesulfonate, thulium(III) trifluoromethanesulfonate, scandium(III) trifluoromethanesulfonate, bismuth(III) trifluoromethanesulfonate, iron(II) trifluoromethanesulfonate, barium(II) trifluoromethanesulfonate, lanthanum(III) chloride bis(lithium chloride) complex, cerium(III) trifluoromethanesulfonate, neodymium(III) trifluoromethanesulfonate, samarium(III) trifluoromethanesulfonate, europium(III) trifluoromethanesulfonate, gadolinium(III) trifluoromethanesulfonate, erbium(III) trifluoromethanesulfonate, lutetium(III) trifluoromethanesulfonate, ytterbium(III) trifluoromethanesulfonate, zinc chloride, indium(III) bromide, vanadium(III) chloride, hafnium(IV) trifluoromethanesulfonate, lanthanum(III) nitrate hexahydrate, and strontium(II) trifluoromethanesulfonate.

4. The method according to any one of claims 1 to 3, wherein the NCA is a compound represented by the following formula (1): 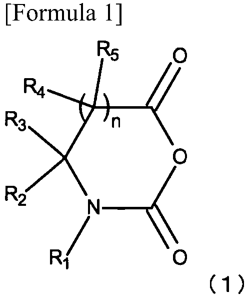
wherein n represents 0 or 1;
(i) R₁ is hydrogen or C₁-C₈ alkyl, or
(ii) R₁ and R₂, together with an atom to which they are bonded, form a 4- to 7-membered saturated heterocycle;
in the case of (i), R₂, R₃, R₄ and R₅ are each independently selected from the group consisting of hydrogen, C₁-C₆ alkyl, C₃-C₈ cycloalkyl, C₆-C₁₀ aryl C₁-C₆ alkyl, and 5- to 10-membered heteroaryl C₁-C₆ alkyl, each of which may be substituted by one or more groups independently selected from the group consisting of halogen, hydroxy, C₁-C₃ alkyl, halo C₁-C₆ alkyl, and C₁-C₆ alkoxycarbonyl, or R₂ and R₃, or R₄ and R₅, together with an atom to which they are each bonded, may form a 3- to 8-membered alicyclic ring or a 4- to 7-membered saturated heterocycle;
in the case of (ii), R₃, R₄, and R₅ are each independently selected from the group consisting of hydrogen, C₁-C₆ alkyl, C₃-C₈ cycloalkyl, C₆-C₁₀ aryl C₁-C₆ alkyl, and 5- to 10-membered heteroaryl C₁-C₆ alkyl, each of which may be substituted by one or more groups independently selected from the group consisting of halogen, hydroxy, C₁-C₃ alkyl, halo C₁-C₆ alkyl, and C₁-C₆ alkoxycarbonyl, or R₄ and R₅, together with an atom to which they are each bonded, may form a 3- to 8-membered alicyclic ring or a 4- to 7-membered saturated heterocycle.

5. The method according to any one of claims 1 to 4, wherein the amino group-containing compound is selected from the group consisting of a peptide, an amino acid, an amino acid amide, and an amine.

6. The method according to any one of claims 1 to 5, wherein the amino group of the amino group-containing compound is a primary amino group or a secondary amino group.

7. The method according to any one of claims 1 to 6, wherein the first solvent is a solvent containing at least one selected from the group consisting of 2-methyltetrahydrofuran, toluene, tetrahydrofuran, 4-methyltetrahydropyran, isopropyl acetate, acetonitrile, and 1,3-dimethyl-2-imidazolidinone.

8. The method according to any one of claims 1 to 7, comprising, prior to the step A, step B of contacting an amino acid protected with a Boc group with a first activator in a second solvent to produce the NCA.

9. The method according to claim 8, comprising, after the step B, step C of removing at least a portion of the second solvent and mixing with a third solvent.

10. The method according to claim 8 or 9, wherein the amino acid protected with a Boc group is a compound represented by the following formula (2): 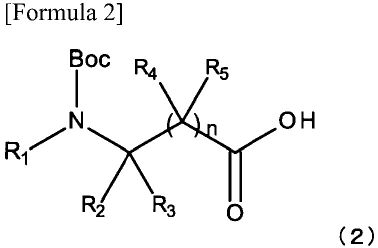 wherein n, R₁, R₂, R₃, R₄ and R₅ has the same meaning as n, R₁, R₂, R₃, R₄ and R₅ in formula (1), respectively.

11. The method according to any one of claims 8 to 10, wherein the second solvent is a solvent containing at least one selected from the group consisting of tetrahydrofuran, 2-methyltetrahydrofuran, isopropyl acetate, ethyl acetate, toluene and acetonitrile.

12. The method according to any one of claims 9 to 11, wherein the third solvent is a solvent containing at least one selected from the group consisting of 2-methyltetrahydrofuran, toluene, tetrahydrofuran, 4-methyltetrahydropyran, isopropyl acetate, acetonitrile, and 1,3-dimethyl-2-imidazolidinone.

13. The method according to any one of claims 8 to 12, wherein the first activator is at least one selected from the group consisting of thionyl chloride, oxalyl chloride, phosphorus trichloride, triphosgene, propylphosphonic anhydride, and dichloromethyl methyl ether.

14. The method according to any one of claims 8 to 13, wherein the step B is carried out in the presence of a base.

15. The method according to claim 14, wherein the base is at least one selected from the group consisting of DIPEA, cesium carbonate, sodium hydrogen carbonate, 1-methylpiperidine, 4-ethylmorpholine, N-methylmorpholine, 2,4,6-collidine, pyridine, potassium carbonate, potassium hydrogen carbonate, magnesium oxide, calcium oxide, tripotassium phosphate, dipotassium hydrogen phosphate, and cesium hydrogen carbonate.

16. The method according to any one of claims 1 to 7, comprising, prior to the step A, step B' of contacting an amino acid with a second activator in a second solvent to produce the NCA.
